# EUROPEAN PATENT APPLICATION

(11) **EP 3 360 555 A1**
(43) Date of publication of application: **15.08.2018**
(21) Application number: 18156337.0
(22) Date of filing: 12.02.2018
(51) Int. Cl.: A61K 31/454, A61K 31/497, A61P 31/22

(54) **METHODS FOR TREATING HCV**

(30) Priority: 14.02.2017 US 201715431906
(71) Applicant: AbbVie Inc., Chicago, IL 60064 (US)
(72) Inventor: AWNI, Walid, Green Oaks, IL Illinois 60048 (US)
(74) Representative: Scheuermann, Erik

(57) **Abstract**

The present invention features interferon-free therapies for the treatment of HCV. Preferably, the treatment is over a shorter duration of treatment, such as no more than 12 weeks. In one aspect, the treatment comprises administering at least two direct acting antiviral agents to a subject with HCV infection, wherein the treatment lasts for 12 weeks and does not include administration of either interferon or ribavirin, and said at least two direct acting antiviral agents comprise (a) Compound 1 or a pharmaceutically acceptable salt thereof and (b) Compound 2 or a pharmaceutically acceptable salt thereof.

## Description

### FIELD OF THE INVENTION

The present invention relates to interferon- and ribavirin-free treatment for hepatitis C virus (HCV).

### BACKGROUND OF THE INVENTION

The HCV is an RNA virus belonging to the Hepacivirus genus in the Flaviviridae family. The enveloped HCV virion contains a positive stranded RNA genome encoding all known virus-specific proteins in a single, uninterrupted, open reading frame. The open reading frame comprises approximately 9500 nucleotides and encodes a single large polyprotein of about 3000 amino acids. The polyprotein comprises a core protein, envelope proteins E1 and E2, a membrane bound protein p7, and the non-structural proteins NS2, NS3, NS4A, NS4B, NS5A and NS5B.

Chronic HCV infection is associated with progressive liver pathology, including cirrhosis and hepatocellular carcinoma. Chronic hepatitis C may be treated with peginterferon-alpha in combination with ribavirin. Substantial limitations to efficacy and tolerability remain as many users suffer from side effects, and viral elimination from the body is often incomplete. Therefore, there is a need for new therapies to treat HCV infection.

### BRIEF SUMMARY OF THE INVENTION

One aspect of the present invention features methods for treating HCV infection in a subject in need of such treatment. The methods comprise administering at least two direct acting antiviral agents (DAAs) to the subject for a duration of no more than 12 weeks, or for another duration as set forth herein. The at least two DAAs comprise Compound 1 (or a pharmaceutically acceptable salt thereof) and Compound 2 (or a pharmaceutically acceptable salt thereof); and the at least two DAAs can also additionally comprise one or more other DAAs, such as sofosbuvir or another HCV polymerase inhibitor. Preferably, the duration of the treatment is 12 weeks. The duration of the treatment can also last for less than 12 weeks; for example, the duration can last for 11, 10, 9, 8, 7, 6, 5 or 4 weeks, or no more than 8 weeks. Where three or more DAAs are used in the treatment regimen, the duration of the treatment preferably lasts for no more than 8 weeks; for example, the duration can last for 8, 7, 6, 5 or 4 weeks. Preferably, the two or more DAAs are administered in amounts effective to provide a sustained virological response (SVR) or achieve another desired measure of effectiveness in the subject. The subject is not administered either interferon or ribavirin during the treatment regimen. Put another way, the methods exclude the administration of interferon and ribavirin to the subject, thereby avoiding the side effects associated with interferon or ribavirin.

Another aspect of the present invention features methods for treating a population of subjects having HCV infection. The methods comprise administering at least two DAAs to the subjects for a duration of no more than 12 weeks, such as for a duration of 12, 11, 10, 9, 8, 7, 6, 5 or 4 weeks, or no more than 8 weeks. The at least two DAAs comprise Compound 1 (or a pharmaceutically acceptable salt thereof) and Compound 2 (or a pharmaceutically acceptable salt thereof); and the at least two DAAs can also additionally comprise one or more other DAAs, such as sofosbuvir or another HCV polymerase inhibitor. Preferably, the at least two DAAs are administered to the subjects in amounts effective to result in SVR or another measure of effectiveness in at least about 70% of the population, preferably at least about 80% of the population, or more preferably at least about 90% of the population.

In any method described herein, the at least two DAAs comprise (a) Compound 1 or a pharmaceutically acceptable salt thereof, and (b) Compound 2 or a pharmaceutically acceptable salt thereof. The at least two DAAs can also optionally comprise one or more other anti-HCV agents. These other optional anti-HCV agents can be selected from protease inhibitors, nucleoside or nucleotide polymerase inhibitors, non-nucleoside polymerase inhibitors, NS3B inhibitors, NS4A inhibitors, NS5A inhibitors, NS5B inhibitors, cyclophilin inhibitors, or combinations thereof. Non-limiting examples of the other optional antic-HCV agents include PSI-7977 (sofosbuvir), PSI-938, BMS-790052 (daclatasvir), BMS-650032 (asunaprevir), BMS-791325, GS-5885 (ledipasvir), GS-9451 (tegobuvir), GS-9190, GS-9256, BI-201335, BI-27127, telaprevir, VX-222, TMC-435 (simepravir), MK-5172, MK-7009 (vaniprevir), danoprevir, R7128 (mericitabine), and any combination thereof.

For example, the DAAs used in a method of the present invention can comprise or consist of (a) Compound 1 or a pharmaceutically acceptable salt thereof, and (b) Compound 2 or a pharmaceutically acceptable salt thereof. For another example, the DAAs used in a method of the present invention can comprise or consist of (a) Compound 1 or a pharmaceutically acceptable salt thereof, (b) Compound 2 or a pharmaceutically acceptable salt thereof, and (c) a HCV polymerase inhibitor, wherein said HCV polymerase inhibitor can be a nucleotide or nucleoside polymerase inhibitor or a non-nucleoside or non-nucleotide polymerase inhibitor. For yet another example, the DAAs used in a method of the present invention can comprise or consist of (a) Compound 1 or a pharmaceutically acceptable salt thereof, (b) Compound 2 or a pharmaceutically acceptable salt thereof, and (c) a nucleotide or nucleoside HCV polymerase inhibitor. For yet another example, the DAAs used in a method of the present invention can comprise or consist of (a) Compound 1 or a pharmaceutically acceptable salt thereof, (b) Compound 2 or a pharmaceutically acceptable salt thereof, and (c) sofosbuvir. For yet another example, the DAAs used in a method of the present invention can comprise or consist of (a) Compound 2 or a pharmaceutically acceptable salt thereof and (b) sofosbuvir.

In any method described herein, the DAAs can be administered in any effective dosing schemes and/or frequencies; for example, they can each be administered daily. Each DAA can be administered either separately or in combination, and each DAA can be administered once a day, twice a day, or three times a day. Preferably, Compound 1 (or a pharmaceutically acceptable salt thereof) and Compound 2 (or a pharmaceutically acceptable salt thereof) are administered once daily (QD).

Preferably, Compound 1 (or a pharmaceutically acceptable salt thereof) is administered from 100 mg to 600 mg once daily, and Compound 2 (or a pharmaceutically acceptable salt thereof) is administered from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. It was unexpectedly found that 200-300 mg Compound 1 has comparable anti-HCV efficacy to 400 mg Compound 1. Therefore, more preferably, Compound 1 (or a pharmaceutically acceptable salt thereof) is administered from 200 mg to 300 mg once daily, and Compound 2 (or a pharmaceutically acceptable salt thereof) is administered from 100 to 500 mg once daily. For example, Compound 1 (or a pharmaceutically acceptable salt thereof) can be administered 200 mg once daily, and Compound 2 (or a pharmaceutically acceptable salt thereof) is administered 120 mg once daily. For another example, Compound 1 (or a pharmaceutically acceptable salt thereof) can be administered 300 mg once daily, and Compound 2 (or a pharmaceutically acceptable salt thereof) is administered 120 mg once daily. For yet another example, Compound 1 (or a pharmaceutically acceptable salt thereof) can be administered 400 mg once daily, and Compound 2 (or a pharmaceutically acceptable salt thereof) is administered 120 mg once daily. For another example, Compound 1 (or a pharmaceutically acceptable salt thereof) can be administered 400 mg once daily, and Compound 2 (or a pharmaceutically acceptable salt thereof) can be administered 240 mg once daily.

In yet another aspect, the present invention features a combination of Compound 1 (or a pharmaceutically acceptable salt thereof) and Compound 2 (or a pharmaceutically acceptable salt thereof) for use to treat HCV infection. The treatment comprises administering the DAAs to a subject infected with HCV. The duration of the treatment regimen is no more than twelve weeks (e.g., the duration being 12 weeks; or the duration being 11, 10, 9, 8, 7, 6, 5, 4, or 3 weeks). Preferably, the duration of the treatment regimen is twelve weeks. The duration of the treatment can also last, for example, no more than eight weeks (e.g., the duration being 8 weeks; or the duration being 7, 6, 5, 4, or 3 weeks). The treatment does not include administering interferon or ribavirin (i.e., neither interferon nor ribavirin are administered). Compound 1 (or the salt thereof) and Compound 2 (or the salt thereof) can be administered concurrently or sequentially. Preferably, Compound 1 (or the salt thereof) and Compound 2 (or the salt thereof) can be administered once daily. As a non-limiting example, the patient being treated is infected with HCV genotype 1, such as genotype 1a or 1b. As another non-limiting example, the patient is infected with HCV genotype 2. As another non-limiting example, the patient is infected with HCV genotype 3. As another non-limiting example, the patient is infected with HCV genotype 4. As another non-limiting example, the patient is infected with HCV genotype 5. As another non-limiting example, the patient is infected with HCV genotype 6. As yet another non-limiting example, the patient is a HCV-treatment naive patient, a HCV-treatment experienced patient, an interferon non-responder (e.g., a null responder), or not a candidate for interferon treatment. As used in this application, the interferon non-responder patients include partial interferon responders and interferon rebound patients. *See* GUIDANCE FOR INDUSTRY - CHRONIC HEPATITIS C VIRUS INFECTION: DEVELOPING DIRECT-ACTING ANTIVIRAL AGENTS FOR TREATMENT (FDA, September 2010, draft guidance) for the definitions of naive, partial responder, responder relapser (i.e., rebound), and null responder patients. The interferon non-responder patients also include null responder patients. In one example of this aspect of the invention, the treatment lasts for 12 weeks, and the subject being treated is a naive patient infected with HCV genotype 1. In another example, the treatment lasts for 11 weeks, and the subject being treated is a naive patient infected with HCV genotype 1. In still another example, the treatment lasts for 10 weeks, and the subject being treated is a naive patient infected with HCV genotype 1. In yet another example, the treatment lasts for 9 weeks, and the subject being treated is a naive patient infected with HCV genotype 1. In yet another example, the treatment lasts for 8 weeks, and the subject being treated is a naive patient infected with HCV genotype 1. In yet another example, the treatment lasts for 7 weeks, and the subject being treated is a naive patient infected with HCV genotype 1. In yet another example, the treatment lasts for 6 weeks, and the subject being treated is a naive patient infected with HCV genotype 1. In yet another example, the treatment lasts for 5 weeks, and the subject being treated is a naive patient infected with HCV genotype 1. In yet another example, the treatment lasts for 4 weeks, and the subject being treated is a naive patient infected with HCV genotype 1. In yet another example, the treatment lasts for 12 weeks, and the subject being treated is a naive patient infected with HCV genotype 3. In another example, the treatment lasts for 11 weeks, and the subject being treated is a naive patient infected with HCV genotype 3. In still another example, the treatment lasts for 10 weeks, and the subject being treated is a naïve patient infected with HCV genotype 3. In yet another example, the treatment lasts for 9 weeks, and the subject being treated is a naive patient infected with HCV genotype 3. In yet another example, the treatment lasts for 8 weeks, and the subject being treated is a naïve patient infected with HCV genotype 3. In yet another example, the treatment lasts for 7 weeks, and the subject being treated is a naive patient infected with HCV genotype 3. In yet another example, the treatment lasts for 6 weeks, and the subject being treated is a naive patient infected with HCV genotype 3. In yet another example, the treatment lasts for 5 weeks, and the subject being treated is a naive patient infected with HCV genotype 3. In yet another example, the treatment lasts for 4 weeks, and the subject being treated is a naive patient infected with HCV genotype 3. In yet another example, the treatment lasts for 12 weeks, and the subject being treated is a non-responder (e.g., a null responder) infected with HCV genotype 1. In another example, the treatment lasts for 11 weeks, and the subject being treated is a non-responder (e.g., a null responder) infected with HCV genotype 1. In still another example, the treatment lasts for 10 weeks, and the subject being treated is a non-responder (e.g., a null responder) infected with HCV genotype 1. In yet another example, the treatment lasts for 9 weeks, and the subject being treated is a non-responder (e.g., a null responder) infected with HCV genotype 1. In yet another example, the treatment lasts for 8 weeks, and the subject being treated is a non-responder (e.g., a null responder) infected with HCV genotype 1. In yet another example, the treatment lasts for 7 weeks, and the subject being treated is a non-responder (e.g., a null responder) infected with HCV genotype 1. In yet another example, the treatment lasts for 6 weeks, and the subject being treated is a non-responder (e.g., a null responder) infected with HCV genotype 1. In yet another example, the treatment lasts for 5 weeks, and the subject being treated is a non-responder (e.g., a null responder) infected with HCV genotype 1. In yet another example, the treatment lasts for 4 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3. In another example, the treatment lasts for 11 weeks, and the subject being treated is a non-responder (e.g., a null responder) infected with HCV genotype 3. In still another example, the treatment lasts for 10 weeks, and the subject being treated is a non-responder (e.g., a null responder) infected with HCV genotype 3. In yet another example, the treatment lasts for 9 weeks, and the subject being treated is a non-responder (e.g., a null responder) infected with HCV genotype 3. In yet another example, the treatment lasts for 8 weeks, and the subject being treated is a non-responder (e.g., a null responder) infected with HCV genotype 3. In yet another example, the treatment lasts for 7 weeks, and the subject being treated is a non-responder (e.g., a null responder) infected with HCV genotype 3. In yet another example, the treatment lasts for 6 weeks, and the subject being treated is a non-responder (e.g., a null responder) infected with HCV genotype 3. In yet another example, the treatment lasts for 5 weeks, and the subject being treated is a non-responder (e.g., a null responder) infected with HCV genotype 3. In yet another example, the treatment lasts for 4 weeks, and the subject being treated is a non-responder (e.g., a null responder) infected with HCV genotype 3.

In yet another aspect, the present invention features a combination of Compound 1 (or a pharmaceutically acceptable salt thereof), Compound 2 (or a pharmaceutically acceptable salt thereof), and an HCV polymerase inhibitor for use to treat HCV infection. The treatment comprises administering the DAAs to a subject infected with HCV. The duration of the treatment regimen is no more than twelve weeks (e.g., the duration being 12 weeks; or the duration being 11, 10, 9, 8, 7, 6, 5, 4, or 3 weeks). Preferably, the duration of the treatment regimen is twelve weeks. The duration of the treatment can also last, for example, no more than eight weeks (e.g., the duration being 8 weeks; or the duration being 7, 6, 5, 4, or 3 weeks). The treatment does not include administering either interferon or ribavirin, i.e., neither interferon nor ribavirin are administered. Compound 1 (or the salt thereof), Compound 2 (or the salt thereof) and the HCV polymerase inhibitor can be administered concurrently or sequentially. Preferably, Compound 1 (or the salt thereof), Compound 2 (or the salt thereof) and the HCV polymerase inhibitor can be administered once daily. As a non-limiting example, the patient being treated is infected with HCV genotype 1, such as genotype 1a or 1b. As another non-limiting example, the patient is infected with HCV genotype 2. As another non-limiting example, the patient is infected with HCV genotype 3. As another non-limiting example, the patient is infected with HCV genotype 4. As another non-limiting example, the patient is infected with HCV genotype 5. As another non-limiting example, the patient is infected with HCV genotype 6. As yet another non-limiting example, the patient is a HCV-treatment naive patient, a HCV-treatment experienced patient, an interferon non-responder (e.g., a null responder), or not a candidate for interferon treatment. In one example of this aspect of the invention, the treatment lasts for 12 weeks, and the subject being treated is a naive patient infected with HCV genotype 1. In another example, the treatment lasts for 11 weeks, and the subject being treated is a naive patient infected with HCV genotype 1. In still another example, the treatment lasts for 10 weeks, and the subject being treated is a naive patient infected with HCV genotype 1. In yet another example, the treatment lasts for 9 weeks, and the subject being treated is a naive patient infected with HCV genotype 1. In yet another example, the treatment lasts for 8 weeks, and the subject being treated is a naive patient infected with HCV genotype 1. In yet another example, the treatment lasts for 7 weeks, and the subject being treated is a naive patient infected with HCV genotype 1. In yet another example, the treatment lasts for 6 weeks, and the subject being treated is a naive patient infected with HCV genotype 1. In yet another example, the treatment lasts for 5 weeks, and the subject being treated is a naive patient infected with HCV genotype 1. In yet another example, the treatment lasts for 4 weeks, and the subject being treated is a naive patient infected with HCV genotype 1. In yet another example, the treatment lasts for 12 weeks, and the subject being treated is a naive patient infected with HCV genotype 3. In another example, the treatment lasts for 11 weeks, and the subject being treated is a naive patient infected with HCV genotype 3. In still another example, the treatment lasts for 10 weeks, and the subject being treated is a naive patient infected with HCV genotype 3. In yet another example, the treatment lasts for 9 weeks, and the subject being treated is a naive patient infected with HCV genotype 3. In yet another example, the treatment lasts for 8 weeks, and the subject being treated is a naive patient infected with HCV genotype 3. In yet another example, the treatment lasts for 7 weeks, and the subject being treated is a naive patient infected with HCV genotype 3. In yet another example, the treatment lasts for 6 weeks, and the subject being treated is a naive patient infected with HCV genotype 3. In yet another example, the treatment lasts for 5 weeks, and the subject being treated is a naive patient infected with HCV genotype 3. In yet another example, the treatment lasts for 4 weeks, and the subject being treated is a naive patient infected with HCV genotype 3. In yet another example, the treatment lasts for 12 weeks, and the subject being treated is a non-responder (e.g., a null responder) infected with HCV genotype 1. In another example, the treatment lasts for 11 weeks, and the subject being treated is a non-responder (e.g., a null responder) infected with HCV genotype 1. In still another example, the treatment lasts for 10 weeks, and the subject being treated is a non-responder (e.g., a null responder) infected with HCV genotype 1. In yet another example, the treatment lasts for 9 weeks, and the subject being treated is a non-responder (e.g., a null responder) infected with HCV genotype 1. In yet another example, the treatment lasts for 8 weeks, and the subject being treated is a non-responder (e.g., a null responder) infected with HCV genotype 1. In yet another example, the treatment lasts for 7 weeks, and the subject being treated is a non-responder (e.g., a null responder) infected with HCV genotype 1. In yet another example, the treatment lasts for 6 weeks, and the subject being treated is a non-responder (e.g., a null responder) infected with HCV genotype 1. In yet another example, the treatment lasts for 5 weeks, and the subject being treated is a non-responder (e.g., a null responder) infected with HCV genotype 1. In yet another example, the treatment lasts for 4 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3. In another example, the treatment lasts for 11 weeks, and the subject being treated is a non-responder (e.g., a null responder) infected with HCV genotype 3. In still another example, the treatment lasts for 10 weeks, and the subject being treated is a non-responder (e.g., a null responder) infected with HCV genotype 3. In yet another example, the treatment lasts for 9 weeks, and the subject being treated is a non-responder (e.g., a null responder) infected with HCV genotype 3. In yet another example, the treatment lasts for 8 weeks, and the subject being treated is a non-responder (e.g., a null responder) infected with HCV genotype 3. In yet another example, the treatment lasts for 7 weeks, and the subject being treated is a non-responder (e.g., a null responder) infected with HCV genotype 3. In yet another example, the treatment lasts for 6 weeks, and the subject being treated is a non-responder (e.g., a null responder) infected with HCV genotype 3. In yet another example, the treatment lasts for 5 weeks, and the subject being treated is a non-responder (e.g., a null responder) infected with HCV genotype 3. In yet another example, the treatment lasts for 4 weeks, and the subject being treated is a non-responder (e.g., a null responder) infected with HCV genotype 3.

In yet another aspect, the present invention features a combination of Compound 1 (or a pharmaceutically acceptable salt thereof), Compound 2 (or a pharmaceutically acceptable salt thereof), and sofosbuvir for use to treat HCV infection. The treatment comprises administering the DAAs to a subject infected with HCV. The duration of the treatment regimen is no more than twelve weeks (e.g., the duration being 12 weeks; or the duration being 11, 10, 9, 8, 7, 6, 5, 4, or 3 weeks). Preferably, the duration of the treatment regimen is twelve weeks. The duration of the treatment can also last, for example, no more than eight weeks (e.g., the duration being 8 weeks; or the duration being 7, 6, 5, 4, or 3 weeks). The treatment does not include administering interferon. Compound 1 (or the salt thereof), Compound 2 (or the salt thereof) and sofosbuvir can be administered concurrently or sequentially. Preferably, Compound 1 (or the salt thereof), Compound 2 (or the salt thereof) and sofosbuvir can be administered once daily. As a non-limiting example, the patient being treated is infected with HCV genotype 1, such as genotype 1a or 1b. As another non-limiting example, the patient is infected with HCV genotype 2. As another non-limiting example, the patient is infected with HCV genotype 3. As another non-limiting example, the patient is infected with HCV genotype 4. As another non-limiting example, the patient is infected with HCV genotype 5. As another non-limiting example, the patient is infected with HCV genotype 6. As yet another non-limiting example, the patient is a HCV-treatment naive patient, a HCV-treatment experienced patient, an interferon non-responder (e.g., a null responder), or not a candidate for interferon treatment. In one example of this aspect of the invention, the treatment lasts for 12 weeks, and the subject being treated is a naive patient infected with HCV genotype 1. In another example, the treatment lasts for 11 weeks, and the subject being treated is a naive patient infected with HCV genotype 1. In still another example, the treatment lasts for 10 weeks, and the subject being treated is a naive patient infected with HCV genotype 1. In yet another example, the treatment lasts for 9 weeks, and the subject being treated is a naive patient infected with HCV genotype 1. In yet another example, the treatment lasts for 8 weeks, and the subject being treated is a naive patient infected with HCV genotype 1. In yet another example, the treatment lasts for 7 weeks, and the subject being treated is a naive patient infected with HCV genotype 1. In yet another example, the treatment lasts for 6 weeks, and the subject being treated is a naive patient infected with HCV genotype 1. In yet another example, the treatment lasts for 5 weeks, and the subject being treated is a naive patient infected with HCV genotype 1. In yet another example, the treatment lasts for 4 weeks, and the subject being treated is a naive patient infected with HCV genotype 1. In yet another example, the treatment lasts for 12 weeks, and the subject being treated is a naive patient infected with HCV genotype 3. In another example, the treatment lasts for 11 weeks, and the subject being treated is a naive patient infected with HCV genotype 3. In still another example, the treatment lasts for 10 weeks, and the subject being treated is a naive patient infected with HCV genotype 3. In yet another example, the treatment lasts for 9 weeks, and the subject being treated is a naive patient infected with HCV genotype 3. In yet another example, the treatment lasts for 8 weeks, and the subject being treated is a naive patient infected with HCV genotype 3. In yet another example, the treatment lasts for 7 weeks, and the subject being treated is a naive patient infected with HCV genotype 3. In yet another example, the treatment lasts for 6 weeks, and the subject being treated is a naive patient infected with HCV genotype 3. In yet another example, the treatment lasts for 5 weeks, and the subject being treated is a naive patient infected with HCV genotype 3. In yet another example, the treatment lasts for 4 weeks, and the subject being treated is a naive patient infected with HCV genotype 3. In yet another example, the treatment lasts for 12 weeks, and the subject being treated is a non-responder (e.g., a null responder) infected with HCV genotype 1. In another example, the treatment lasts for 11 weeks, and the subject being treated is a non-responder (e.g., a null responder) infected with HCV genotype 1. In still another example, the treatment lasts for 10 weeks, and the subject being treated is a non-responder (e.g., a null responder) infected with HCV genotype 1. In yet another example, the treatment lasts for 9 weeks, and the subject being treated is a non-responder (e.g., a null responder) infected with HCV genotype 1. In yet another example, the treatment lasts for 8 weeks, and the subject being treated is a non-responder (e.g., a null responder) infected with HCV genotype 1. In yet another example, the treatment lasts for 7 weeks, and the subject being treated is a non-responder (e.g., a null responder) infected with HCV genotype 1. In yet another example, the treatment lasts for 6 weeks, and the subject being treated is a non-responder (e.g., a null responder) infected with HCV genotype 1. In yet another example, the treatment lasts for 5 weeks, and the subject being treated is a non-responder (e.g., a null responder) infected with HCV genotype 1. In yet another example, the treatment lasts for 4 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3. In another example, the treatment lasts for 11 weeks, and the subject being treated is a non-responder (e.g., a null responder) infected with HCV genotype 3. In still another example, the treatment lasts for 10 weeks, and the subject being treated is a non-responder (e.g., a null responder) infected with HCV genotype 3. In yet another example, the treatment lasts for 9 weeks, and the subject being treated is a non-responder (e.g., a null responder) infected with HCV genotype 3. In yet another example, the treatment lasts for 8 weeks, and the subject being treated is a non-responder (e.g., a null responder) infected with HCV genotype 3. In yet another example, the treatment lasts for 7 weeks, and the subject being treated is a non-responder (e.g., a null responder) infected with HCV genotype 3. In yet another example, the treatment lasts for 6 weeks, and the subject being treated is a non-responder (e.g., a null responder) infected with HCV genotype 3. In yet another example, the treatment lasts for 5 weeks, and the subject being treated is a non-responder (e.g., a null responder) infected with HCV genotype 3. In yet another example, the treatment lasts for 4 weeks, and the subject being treated is a non-responder (e.g., a null responder) infected with HCV genotype 3.

In yet another aspect, the present invention features a combination of Compound 2 (or a pharmaceutically acceptable salt thereof) and sofosbuvir for use to treat HCV infection. The treatment comprises administering the DAAs to a subject infected with HCV. The duration of the treatment regimen is no more than twelve weeks (e.g., the duration being 12 weeks; or the duration being 11, 10, 9, 8, 7, 6, 5, 4, or 3 weeks). Preferably, the duration of the treatment regimen is twelve weeks. The duration of the treatment can also last, for example, no more than eight weeks (e.g., the duration being 8 weeks; or the duration being 7, 6, 5, 4, or 3 weeks). The treatment does not include administering interferon. Compound 2 (or the salt thereof) and sofosbuvir can be administered concurrently or sequentially. Preferably, Compound 2 (or the salt thereof) and sofosbuvir can be administered once daily. As a non-limiting example, the patient being treated is infected with HCV genotype 1, such as genotype 1a or 1b. As another non-limiting example, the patient is infected with HCV genotype 2. As another non-limiting example, the patient is infected with HCV genotype 3. As another non-limiting example, the patient is infected with HCV genotype 4. As another non-limiting example, the patient is infected with HCV genotype 5. As another non-limiting example, the patient is infected with HCV genotype 6. As yet another non-limiting example, the patient is a HCV-treatment naive patient, a HCV-treatment experienced patient, an interferon non-responder (e.g., a null responder), or not a candidate for interferon treatment. In one example of this aspect of the invention, the treatment lasts for 12 weeks, and the subject being treated is a naive patient infected with HCV genotype 1. In another example, the treatment lasts for 11 weeks, and the subject being treated is a naive patient infected with HCV genotype 1. In still another example, the treatment lasts for 10 weeks, and the subject being treated is a naive patient infected with HCV genotype 1. In yet another example, the treatment lasts for 9 weeks, and the subject being treated is a naive patient infected with HCV genotype 1. In yet another example, the treatment lasts for 8 weeks, and the subject being treated is a naive patient infected with HCV genotype 1. In yet another example, the treatment lasts for 7 weeks, and the subject being treated is a naive patient infected with HCV genotype 1. In yet another example, the treatment lasts for 6 weeks, and the subject being treated is a naive patient infected with HCV genotype 1. In yet another example, the treatment lasts for 5 weeks, and the subject being treated is a naive patient infected with HCV genotype 1. In yet another example, the treatment lasts for 4 weeks, and the subject being treated is a naive patient infected with HCV genotype 1. In yet another example, the treatment lasts for 12 weeks, and the subject being treated is a naive patient infected with HCV genotype 3. In another example, the treatment lasts for 11 weeks, and the subject being treated is a naive patient infected with HCV genotype 3. In still another example, the treatment lasts for 10 weeks, and the subject being treated is a naive patient infected with HCV genotype 3. In yet another example, the treatment lasts for 9 weeks, and the subject being treated is a naive patient infected with HCV genotype 3. In yet another example, the treatment lasts for 8 weeks, and the subject being treated is a naive patient infected with HCV genotype 3. In yet another example, the treatment lasts for 7 weeks, and the subject being treated is a naive patient infected with HCV genotype 3. In yet another example, the treatment lasts for 6 weeks, and the subject being treated is a naive patient infected with HCV genotype 3. In yet another example, the treatment lasts for 5 weeks, and the subject being treated is a naive patient infected with HCV genotype 3. In yet another example, the treatment lasts for 4 weeks, and the subject being treated is a naive patient infected with HCV genotype 3. In yet another example, the treatment lasts for 12 weeks, and the subject being treated is a non-responder (e.g., a null responder) infected with HCV genotype 1. In another example, the treatment lasts for 11 weeks, and the subject being treated is a non-responder (e.g., a null responder) infected with HCV genotype 1. In still another example, the treatment lasts for 10 weeks, and the subject being treated is a non-responder (e.g., a null responder) infected with HCV genotype 1. In yet another example, the treatment lasts for 9 weeks, and the subject being treated is a non-responder (e.g., a null responder) infected with HCV genotype 1. In yet another example, the treatment lasts for 8 weeks, and the subject being treated is a non-responder (e.g., a null responder) infected with HCV genotype 1. In yet another example, the treatment lasts for 7 weeks, and the subject being treated is a non-responder (e.g., a null responder) infected with HCV genotype 1. In yet another example, the treatment lasts for 6 weeks, and the subject being treated is a non-responder (e.g., a null responder) infected with HCV genotype 1. In yet another example, the treatment lasts for 5 weeks, and the subject being treated is a non-responder (e.g., a null responder) infected with HCV genotype 1. In yet another example, the treatment lasts for 4 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3. In another example, the treatment lasts for 11 weeks, and the subject being treated is a non-responder (e.g., a null responder) infected with HCV genotype 3. In still another example, the treatment lasts for 10 weeks, and the subject being treated is a non-responder (e.g., a null responder) infected with HCV genotype 3. In yet another example, the treatment lasts for 9 weeks, and the subject being treated is a non-responder (e.g., a null responder) infected with HCV genotype 3. In yet another example, the treatment lasts for 8 weeks, and the subject being treated is a non-responder (e.g., a null responder) infected with HCV genotype 3. In yet another example, the treatment lasts for 7 weeks, and the subject being treated is a non-responder (e.g., a null responder) infected with HCV genotype 3. In yet another example, the treatment lasts for 6 weeks, and the subject being treated is a non-responder (e.g., a null responder) infected with HCV genotype 3. In yet another example, the treatment lasts for 5 weeks, and the subject being treated is a non-responder (e.g., a null responder) infected with HCV genotype 3. In yet another example, the treatment lasts for 4 weeks, and the subject being treated is a non-responder (e.g., a null responder) infected with HCV genotype 3.

A treatment regimen of the present invention generally constitutes a complete treatment regimen, i.e., no subsequent interferon-containing regimen is intended. Thus, a treatment or use described herein generally does not include any subsequent interferon-containing or ribavirin-containing treatment.

Other features, objects, and advantages of the present invention are apparent in the detailed description that follows. It should be understood, however, that the detailed description, while indicating preferred embodiments of the invention, are given by way of illustration only, not limitation. Various changes and modifications within the scope of the invention will become apparent to those skilled in the art from the detailed description

### BRIEF DESCRIPTION OF THE DRAWINGS

The drawings are provided for illustration, not limitation.
Figure 1 shows the predicted median SVR percentages and 90% SVR confidence intervals for interferon/ribavirin-free, 2-DAA regimens comprising the use of Compound 1 (400 mg once daily) and Compound 2 (120 mg once daily) to treat genotype 1 naive subjects.
Figure 2 illustrates the predicted median SVR percentages and 90% SVR confidence intervals for interferon/ribavirin-free, 2-DAA regimens comprising the use of Compound 1 (400 mg once daily) and Compound 2 (60 mg once daily) to treat genotype 1 naive subjects.
Figure 3 depicts the predicted median SVR percentages and 90% SVR confidence intervals for interferon/ribavirin-free, 2-DAA regimens comprising the use of Compound 1 (600 mg once daily) and Compound 2 (480 mg once daily) to treat genotype 1 naive subjects.
Figure 4 shows the predicted median SVR percentages and 90% SVR confidence intervals for interferon/ribavirin-free, 2-DAA regimens comprising the use of Compound 1 (400 mg once daily) and Compound 2 (120 mg once daily) to treat genotype 3 naive subjects.
Figure 5 illustrates the predicted median SVR percentages and 90% SVR confidence intervals for interferon/ribavirin-free, 2-DAA regimens comprising the use of Compound 1 (400 mg once daily) and Compound 2 (60 mg once daily) to treat genotype 3 naive subjects.
Figure 6 shows the predicted median SVR percentages and 90% SVR confidence intervals for interferon/ribavirin-free, 2-DAA regimens comprising the use of Compound 1 (600 mg once daily) and Compound 2 (480 mg once daily) to treat genotype 3 naive subjects.
Figure 7 shows the predicted median SVR percentages and 90% SVR confidence intervals for interferon/ribavirin-free, 3-DAA regimens comprising the use of Compound 1 (400 mg once daily), Compound 2 (120 mg once daily) and sofosbuvir (400 mg once daily) to treat genotype 1 naive subjects.
Figure 8 shows the predicted median SVR percentages and 90% SVR confidence intervals for interferon/ribavirin-free, 2-DAA regimens comprising the use of Compound 2 (120 mg once daily) and sofosbuvir (400 mg once daily) to treat genotype 1 naive subjects.
Figure 9 depict the synergistic effect of the combination of Compound 1 and Compound 2 on HCV inhibition *in vitro.*

### DETAILED DESCRIPTION OF THE INVENTION

The methods of the present invention include administering Compound 1 (or a pharmaceutically acceptable salt thereof) and Compound 2 (or a pharmaceutically acceptable salt thereof) to a subject in need thereof. Compound 1 has the following structure: 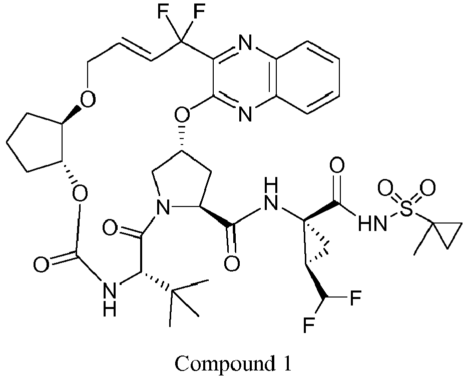 Compound 1 is a potent HCV protease inhibitor and is described in U.S. Patent Application Publication No. 2012/0070416.

Compound 2 has the following structure: 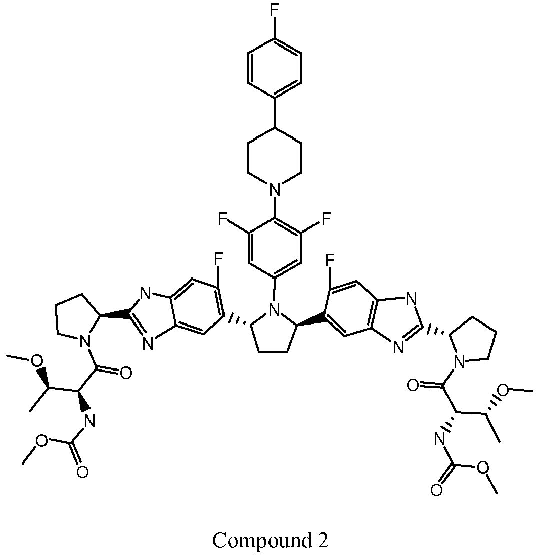 Compound 2 is a potent NS5A inhibitor and is described in U.S. Patent Application Publication No. 2012/0220562.

The interferon/ribavirin-based treatment may be physically demanding, and can lead to temporary disability in some cases. A substantial proportion of patients will experience a panoply of side effects ranging from a "flu-like" syndrome (the most common, experienced for a few days after the weekly injection of interferon) to severe adverse events including anemia, cardiovascular events and psychiatric problems such as suicide or suicidal ideation. The latter are exacerbated by the general physiological stress experienced by the patients. Ribavirin also has a number of side effects, including, anemia, high pill burden (e.g. 5-6 pills a day split BID) and teratogenicity restricting use in women of childbearing age.

The methods of the present invention provide effective treatment of HCV infection without the use of interferon or ribavirin and for a shorter period of time, for example and without limitation, a treatment duration of no more than twelve weeks, alternatively no more than eleven weeks, alternatively no more than ten weeks, alternatively no more than nine weeks, alternatively no more than eight weeks, alternatively no more than seven weeks, alternatively no more than six weeks, alternatively no more than five weeks, alternatively no more than four weeks, or alternatively, no more than three weeks.

In one aspect, the present invention features methods for treating HCV infection in a subject comprising administering at least two DAAs, in the absence of interferon and ribavirin, to the subject for a duration of no more than twelve weeks, alternatively no more than eight weeks, such as for a duration of 12, 11, 10, 9, 8, 7, 6, 5, or 4 weeks. Put another way, the methods exclude interferon and ribavirin, i.e. neither interferon nor ribavirin are administered. The at least two DAAs comprise Compound 1 (or a pharmaceutically acceptable salt thereof) and Compound 2 (or a pharmaceutically acceptable salt thereof), which can be co-administered, or administered separately or independently, with the same or different dosing frequencies. Preferably, the at least two DAAs are administered once a day. They can also be administered, for example, twice a day or three times a day.

In one aspect, the present invention features methods for treating HCV infection in a subject comprising administering at least two DAAs, in the absence of interferon and ribavirin, to the subject for a duration of no more than twelve weeks, alternatively no more than eight weeks, such as for a duration of 12, 11, 10, 9, 8, 7, 6, 5, or 4 weeks. Put another way, the methods exclude interferon and ribavirin, i.e., neither interferon nor ribavirin are administered. The at least two DAAs comprise Compound 1 (or a pharmaceutically acceptable salt thereof), Compound 2 (or a pharmaceutically acceptable salt thereof) and an HCV polymerase inhibitor, which can be co-administered, or administered separately or independently, with the same or different dosing frequencies. Preferably, the at least two DAAs are administered once a day. They can also be administered, for example, twice a day or three times a day.

In one aspect, the present invention features methods for treating HCV infection in a subject comprising administering at least two DAAs, in the absence of interferon and ribavirin, to the subject for a duration of no more than twelve weeks, alternatively no more than eight weeks, such as for a duration of 12, 11, 10, 9, 8, 7, 6, 5, or 4 weeks. Put another way, the methods exclude interferon and ribavirin, i.e., neither interferon nor ribavirin are administered. The at least two DAAs comprise Compound 1 (or a pharmaceutically acceptable salt thereof), Compound 2 (or a pharmaceutically acceptable salt thereof) and sofosbuvir, which can be co-administered, or administered separately or independently, with the same or different dosing frequencies. Preferably, the at least two DAAs are administered once a day. They can also be administered, for example, twice a day or three times a day.

In one aspect, the present invention features methods for treating HCV infection in a subject comprising administering at least two DAAs, in the absence of interferon and ribavirin, to the subject for a duration of no more than twelve weeks, alternatively no more than eight weeks, such as for a duration of 12, 11, 10, 9, 8, 7, 6, 5, or 4 weeks. Put another way, the methods exclude interferon and ribavirin, i.e., neither interferon nor ribavirin are administered. The at least two DAAs comprise Compound 2 (or a pharmaceutically acceptable salt thereof) and sofosbuvir, which can be co-administered, or administered separately or independently, with the same or different dosing frequencies. Preferably, the at least two DAAs are administered once a day. They can also be administered, for example, twice a day or three times a day.

Various measures may be used to express the effectiveness of a method of the present invention. One such measure is SVR, which, as used herein, means that the virus is undetectable at the end of therapy and for at least 8 weeks after the end of therapy (SVR8); preferably, the virus is undetectable at the end of therapy and for at least 12 weeks after the end of therapy (SVR12); more preferably, the virus is undetectable at the end of therapy and for at least 16 weeks after the end of therapy (SVR16); and highly preferably, the virus is undetectable at the end of therapy and for at least 24 weeks after the end of therapy (SVR24). SVR24 is often considered as a functional definition of cure; and a high rate of SVR at less than 24 week post-treatment (e.g., SVR8 or SVR12) can be predictive of a high rate of SVR24.

Preferably, a method described herein achieves at least 70% SVR8. More preferably, a method described herein achieves at least 80% SVR8. Highly preferably, a method described herein achieves at least 90% SVR8. Most preferably, a method described herein achieves at least 95% SVR8.

Preferably, a method described herein achieves at least 70% SVR12. More preferably, a method described herein achieves at least 80% SVR12. Highly preferably, a method described herein achieves at least 90% SVR12. Most preferably, a method described herein achieves at least 95% SVR12. A method without achieving a significant SVR rate within patients is not considered an effective treatment, despite the fact that other effectiveness measures (e.g., RVR, eRVR, EVR, or ETR) may show suppression of the HCV virus during the treatment or immediately at the end of the treatment.

In some embodiments, a treatment regimen of the invention comprises treating a population of subjects having HCV infection (e.g. treatment naive subjects), and the regimen comprises administering at least two DAAs to the subjects for a duration of no more than 12 weeks, or for another duration disclosed herein (e.g., 11, 10, 9, 8, 7, 6, 5, or 4 weeks), wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs to the subjects for a duration of no more than 12 weeks, or for another duration disclosed herein, wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

In some embodiments, a treatment regimen of the invention comprises treating a population of subjects having HCV infection (e.g. treatment naive subjects), and the regimen comprises administering at least two DAAs to the subjects for a duration of no more than 12 weeks, or for another duration disclosed herein (e.g., 11, 10, 9, 8, 7, 6, 5, or 4 weeks), wherein the at least two DAAs comprise Compound 1 (or a pharmaceutically acceptable salt thereof), Compound 2 (or a pharmaceutically acceptable salt thereof) and an HCV polymerase inhibitor, and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs to the subjects for a duration of no more than 12 weeks, or for another duration disclosed herein, wherein the at least two DAAs comprise Compound 1 (or a pharmaceutically acceptable salt thereof), Compound 2 (or a pharmaceutically acceptable salt thereof) and an HCV polymerase inhibitor, and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

In some embodiments, a treatment regimen of the invention comprises treating a population of subjects having HCV infection (e.g. treatment naive subjects), and the regimen comprises administering at least two DAAs to the subjects for a duration of no more than 12 weeks, or for another duration disclosed herein (e.g., 11, 10, 9, 8, 7, 6, 5, or 4 weeks), wherein the at least two DAAs comprise Compound 1 (or a pharmaceutically acceptable salt thereof), Compound 2 (or a pharmaceutically acceptable salt thereof) and sofosbuvir, and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs to the subjects for a duration of no more than 12 weeks, or for another duration disclosed herein, wherein the at least two DAAs comprise Compound 1 (or a pharmaceutically acceptable salt thereof), Compound 2 (or a pharmaceutically acceptable salt thereof) and sofosbuvir, and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

In some embodiments, a treatment regimen of the invention comprises treating a population of subjects having HCV infection (e.g. treatment naive subjects), and the regimen comprises administering at least two DAAs to the subjects for a duration of no more than 12 weeks, or for another duration disclosed herein (e.g., 11, 10, 9, 8, 7, 6, 5, or 4 weeks), wherein the at least two DAAs comprise Compound 2 (or a pharmaceutically acceptable salt thereof) and sofosbuvir, and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs to the subjects for a duration of no more than 12 weeks, or for another duration disclosed herein, wherein the at least two DAAs comprise Compound 2 (or a pharmaceutically acceptable salt thereof) and sofosbuvir, and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

It was unexpected that an interferon-free treatment using a combination of Compound 1 (or a pharmaceutically acceptable salt thereof) and Compound 2 (or a pharmaceutically acceptable salt thereof), in the absence of interferon and ribavirin, and for a duration of no more than 12 weeks, can achieve significant SVR.

Accordingly, in one aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 8 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 7 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 6 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 5 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 4 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 3 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 24 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 13 to 23 weeks (e.g., the duration of the treatment is selected from 13, 14, 15, 16, 17, 18, 19, 20, 21, 22 or 23 weeks) and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 12 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

As used in this application, an HCV polymerase inhibitor can be a nucleoside polymerase inhibitor, a nucleotide polymerase inhibitor, a non-nucleoside polymerase inhibitor, or a non-nucleotide polymerase inhibitor.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 11 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 10 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 9 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 8 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 7 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 6 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 5 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 4 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 3 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 24 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 13 to 23 weeks (e.g., the duration of the treatment is selected from 13, 14, 15, 16, 17, 18, 19, 20, 21, 22 or 23 weeks) and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 12 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 11 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 10 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and an HCV polymerase inhibitor. The treatment lasts 9 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and the HCV polymerase inhibitor, said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 8 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 7 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 6 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 5 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 4 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 3 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 24 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 13 to 23 weeks (e.g., the duration of the treatment is selected from 13, 14, 15, 16, 17, 18, 19, 20, 21, 22 or 23 weeks) and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 12 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 11 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 10 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof), Compound 2 (or a pharmaceutically acceptable salt thereof) and sofosbuvir. The treatment lasts 9 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof), Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 8 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 7 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 6 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 5 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 4 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 3 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 24 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 13 to 23 weeks (e.g., the duration of the treatment is selected from 13, 14, 15, 16, 17, 18, 19, 20, 21, 22 or 23 weeks) and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 12 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 11 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 10 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 2 (or a pharmaceutically acceptable salt thereof) and sofosbuvir. The treatment lasts 9 weeks and does not include administration of any interferon or ribavirin (i.e., neither interferon nor ribavirin are administered). The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 2 (or a salt thereof) and sofosbuvir, said at least two DAAs can also include one or more additional DAAs selected from, for example, HCV protease inhibitors, HCV polymerase inhibitors, or HCV NS5A inhibitors. Non-limiting examples of such additional DAAs include PSI-938, TMC-435, BMS-790052, BMS-650032, GS-5885, GS-9190, GS-9451, BI-201335, BI-207127, telaprevir, VX-222, mericitabine, and danoprevir.

In each aspect, embodiment, example or method described herein, Compound 1 (or a pharmaceutically acceptable salt thereof) can be administered, for example and without limitation, from 100 mg to 600 mg once daily, and Compound 2 (or a pharmaceutically acceptable salt thereof) can be administered, for example and without limitation, from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. Most preferably, Compound 1 (or a pharmaceutically acceptable salt thereof) is administered from 200 mg to 300 mg once daily, and Compound 2 (or a pharmaceutically acceptable salt thereof) is administered from 100 to 500 mg once daily. Preferably, Compound 1 (or a pharmaceutically acceptable salt thereof) can be administered 200 mg once daily, and Compound 2 (or a pharmaceutically acceptable salt thereof) is administered 120 mg once daily. Also preferably, Compound 1 (or a pharmaceutically acceptable salt thereof) can be administered 300 mg once daily, and Compound 2 (or a pharmaceutically acceptable salt thereof) is administered 120 mg once daily. For another example, Compound 1 (or a pharmaceutically acceptable salt thereof) can be administered 400 mg once daily, and Compound 2 (or a pharmaceutically acceptable salt thereof) is administered 120 mg once daily. For yet another example, Compound 1 (or a pharmaceutically acceptable salt thereof) can be administered 400 mg once daily, and Compound 2 (or a pharmaceutically acceptable salt thereof) can be administered 240 mg once daily.

In each aspect, embodiment, example or method described herein, sofosbuvir can be administered, for example and without limitation, 400 mg once daily.

A method of the present invention can be used to treat a naive patient or a treatment experienced patient. Treatment experienced patients include interferon non-responders (e.g., null responders), partial responders, and relapsers. A method of the present invention can also be used to treat patients who are not candidates for interferon treatment. Patients who are not candidates for interferon treatment include, but are not limited to, one or more of the following groups: patients intolerant to interferon, patients who refuse to take interferon treatment, patients with medical conditions which preclude them from taking interferon, and patients who have an increased risk of side effects or infection by taking interferon.

In any method described herein wherein Compound 1 and Compound 2 are used, one or more additional DAAs can be optionally used in the treatment regimen in addition to Compound 1 (or a salt thereof) and Compound 2 (or a salt thereof). Similarly, in any method described herein wherein Compound 1, Compound 2 and sofosbuvir are used, one or more additional DAAs can be optionally used in the treatment regimen in addition to Compound 1 (or a salt thereof), Compound 2 (or a salt thereof) and sofosbuvir. Likewise, in any method described herein wherein Compound 2 and sofosbuvir are used, one or more additional DAAs can be optionally used in the treatment regimen in addition to Compound 2 (or a salt thereof) and sofosbuvir. These additional DAAs can be HCV protease inhibitors, HCV nucleoside or nucleotide polymerase inhibitors, HCV non-nucleoside polymerase inhibitors, HCV NS3B inhibitors, HCV NS4A inhibitors, HCV NS5A inhibitors, HCV NS5B inhibitors, HCV entry inhibitors, cyclophilin inhibitors, or combinations thereof.

Preferred HCV protease inhibitors for this purpose include, but are not limited to, telaprevir (Vertex), boceprevir (Merck), BI-201335 (Boehringer Ingelheim), GS-9451 (Gilead), and BMS-650032 (BMS). Other suitable protease inhibitors include, but are not limited to, ACH-1095 (Achillion), ACH-1625 (Achillion), ACH-2684 (Achillion), AVL-181 (Avila), AVL-192 (Avila), BMS-650032 (BMS), danoprevir (RG7227/ITMN-191, Roche), GS-9132 (Gilead), GS-9256 (Gilead), IDX-136 (Idenix), IDX-316 (Idenix), IDX-320 (Idenix), MK-5172 (Merck), narlaprevir (Schering-Plough Corp), PHX-1766 (Phenomix), TMC-435 (Tibotec), vaniprevir (MK-7009, Merck), VBY708 (Virobay), VX-500 (Vertex), VX-813 (Vertex), VX-985 (Vertex), or a combination thereof.

Preferred non-nucleoside HCV polymerase inhibitors for use in the present invention include, but are not limited to, GS-9190 (Gilead), BI-207127 (Boehringer Ingelheim), and VX-222 (VCH-222) (Vertex & ViraChem). Preferred nucleotide HCV polymerase inhibitors include, but are not limited to, PSI-7977 (Gilead), and PSI-938 (Gilead). Other suitable and non-limiting examples of suitable HCV polymerase inhibitors include ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir, TMC-647055 (Tibotec), VCH-759 (Vertex & ViraChem), VCH-916 (ViraChem), VX-759 (Vertex), GS-6620 (Gilead), IDX-102 (Idenix), IDX-184 (Idenix), INX-189 (Inhibitex), MK-0608 (Merck), RG7128 (Roche), TMC64912 (Medivir), GSK625433 (GlaxoSmithKline), BCX-4678 (BioCryst), ALS-2200 (Alios BioPharma/Vertex), ALS-2158 (Alios BioPharma/Vertex), or a combination thereof. A polymerase inhibitor may be a nucleoside or nucleotide polymerase inhibitor, such as GS-6620 (Gilead), IDX-102 (Idenix), IDX-184 (Idenix), INX-189 (Inhibitex), MK-0608 (Merck), PSI-7977 (Gilead), PSI-938 (Gilead), RG7128 (Roche), TMC64912 (Medivir), ALS-2200 (Alios BioPharma/Vertex), ALS-2158 (Alios BioPharma/Vertex), or a combination therefore. A polymerase inhibitor may also be a non-nucleoside polymerase inhibitor, such as PF-00868554 (Pfizer), ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir (Gilead),, TMC-647055 (Tibotec), VCH-759 (Vertex & ViraChem), VCH-916 (ViraChem), VX-222 (VCH-222) (Vertex & ViraChem), VX-759 (Vertex), or a combination thereof.

Preferred NS5A inhibitors include, but are not limited to, BMS-790052 (BMS) and GS-5885 (Gilead). Non-limiting examples of suitable NS5A inhibitors include GSK62336805 (GlaxoSmithKline), ACH-2928 (Achillion), AZD2836 (Astra-Zeneca), AZD7295 (Astra-Zeneca), BMS-790052 (BMS), BMS-824393 (BMS), GS-5885 (Gilead), PPI-1301 (Presidio), PPI-461 (Presidio) A-831 (Arrow Therapeutics), A-689 (Arrow Therapeutics) or a combination thereof.

Non-limiting examples of suitable cyclophilin inhibitors include alisporovir (Novartis & Debiopharm), NM-811 (Novartis), SCY-635 (Scynexis), or a combination thereof.

Non-limiting examples of suitable HCV entry inhibitors include ITX-4520 (iTherx), ITX-5061 (iTherx), or a combination thereof.

Specific examples of other DAA agents that are suitable for inclusion in a method of the present invention include, but are not limited to, AP-H005, A-831 (Arrow Therapeutics) (NS5A inhibitor), A-689 (Arrow Therapeutics) (NS5A inhibitor), INX08189 (Inhibitex) (polymerase inhibitor), ITMN-191 (Intermune/Roche) (NS3/4A Protease inhibitor), VBY-376 (Protease Inhibitor) (Virobay), ACH-1625 (Achillion, Protease inhibitor), IDX136 (Idenix, Protease Inhibitor), IDX316 (Idenix, Protease inhibitor), VX-813 (Vertex), SCH 900518 (Schering-Plough), TMC-435 (Tibotec), ITMN-191 (Intermune, Roche), MK-7009 (Merck), IDX-PI (Novartis), R7128 (Roche), PF-868554 (Pfizer) (non-nucleoside polymerase inhibitor), PF-4878691 (Pfizer), IDX-184 (Idenix), IDX-375 (Idenix, NS5B polymerase inhibitor), PPI-461 (Presidio), BILB-1941 (Boehringer Ingelheim), GS-9190 (Gilead), BMS-790052 (BMS), CTS-1027 (Conatus), GS-9620 (Gilead), PF-4878691 (Pfizer), RO5303253 (Roche), ALS-2200 (Alios BioPharma/Vertex), ALS-2158 (Alios BioPharma/Vertex), GSK62336805 (GlaxoSmithKline), or any combinations thereof.

The chemical structures of some of these optional HCV inhibitors are provided below:

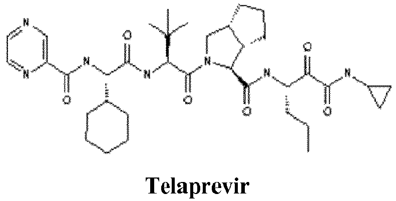

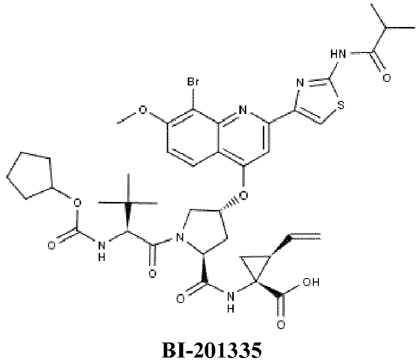

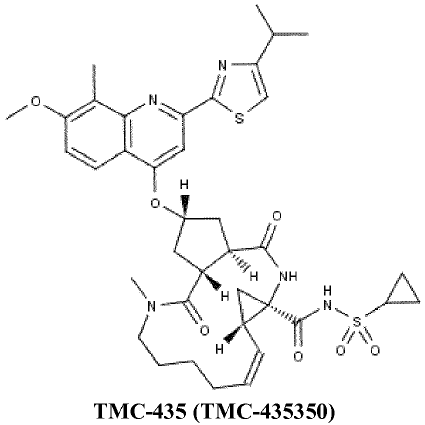

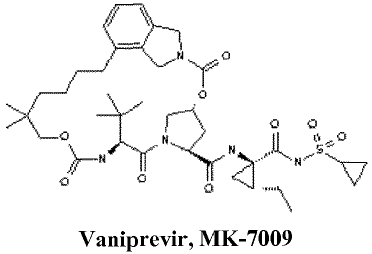

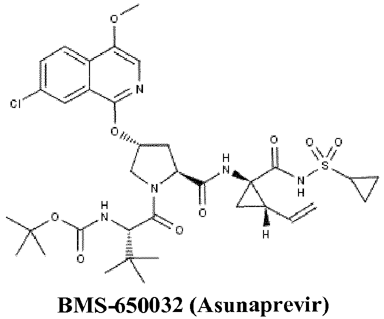

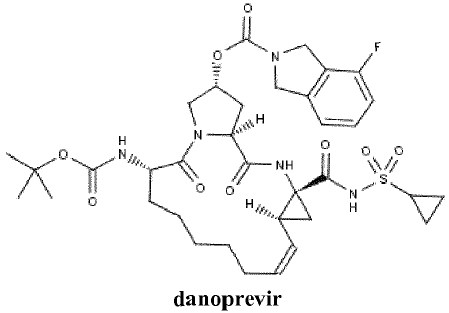

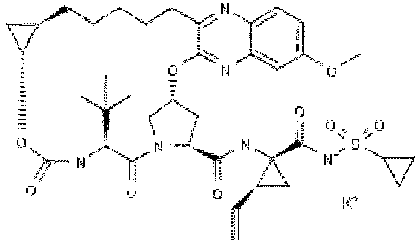

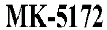

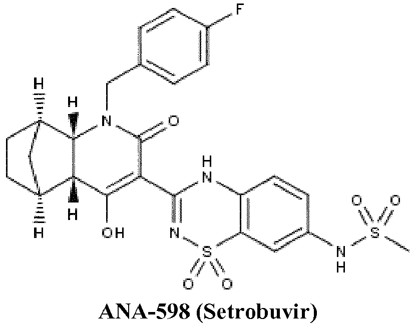

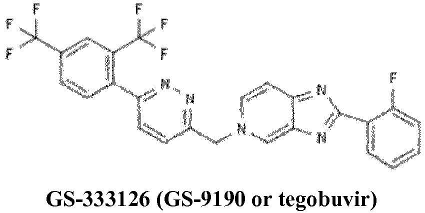

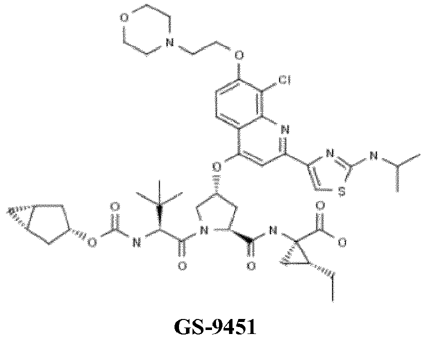

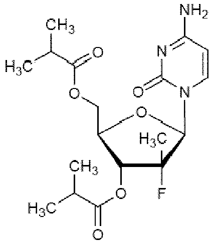

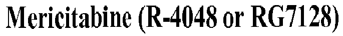

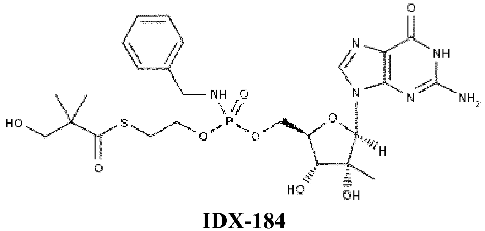

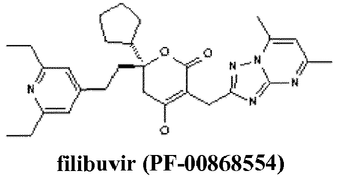

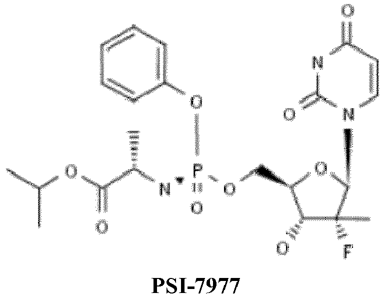

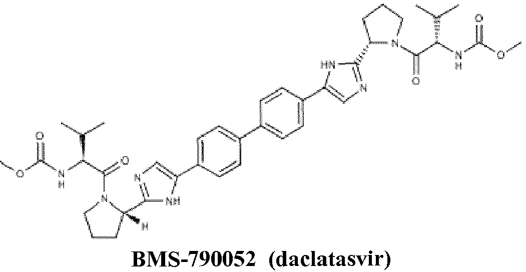

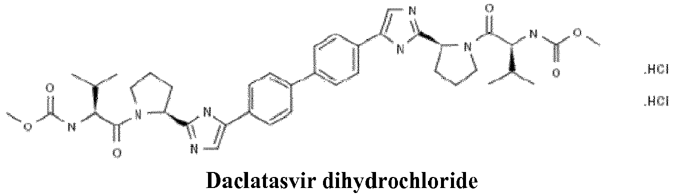

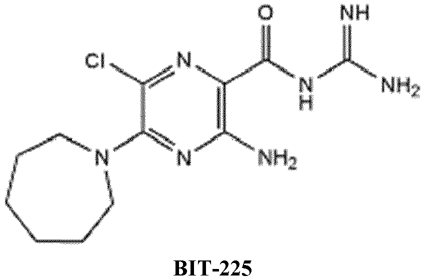

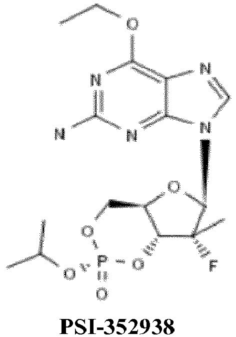

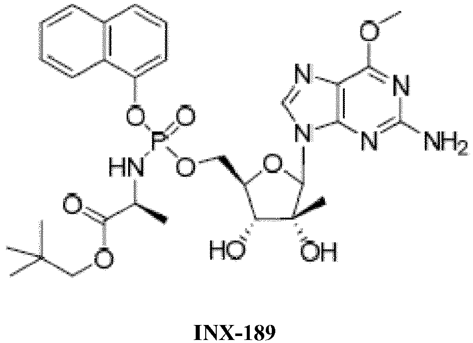

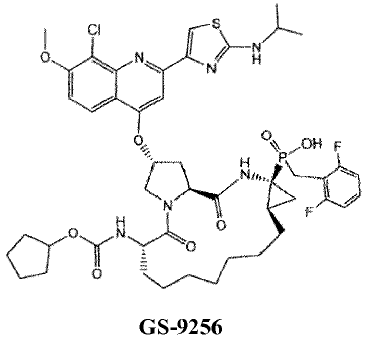

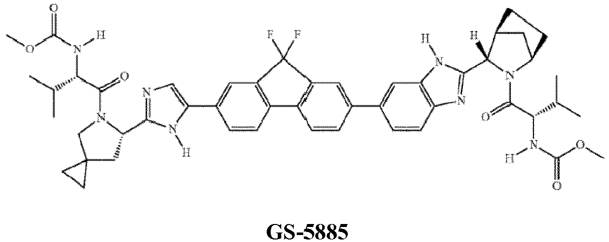

Any HCV inhibitor or DAA described herein encompasses its suitable salt forms when it is used in therapeutic treatments or pharmaceutical formulations.

In some embodiments, the present invention features methods for treating patients infected with HCV genotype 1, such as 1a or 1b. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 or 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 4 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 5 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 6 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients infected with HCV genotype 1, such as 1a or 1b. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin (i.e., neither interferon nor ribavirin are administered), and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 or 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 4 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 5 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 6 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and an HCV polymerase inhibitor. Compound 1 (or a pharmaceutically acceptable salt thereof), Compound 2 (a pharmaceutically acceptable salt thereof) and the HCV polymerase inhibitor can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients infected with HCV genotype 1, such as 1a or 1b. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 or 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 4 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 5 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 6 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 1 (or a pharmaceutically acceptable salt thereof), Compound 2 (a pharmaceutically acceptable salt thereof) and sofosbuvir. Compound 1 (or a pharmaceutically acceptable salt thereof), Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients infected with HCV genotype 1, such as 1a or 1b. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 or 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 2 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 3 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 4 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 5 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

In some embodiments, the present invention features methods for treating patients with HCV genotype 6 infection. The methods comprise administering to such a patient a combination of at least 2 DAAs for no more than 12 weeks (e.g., the duration being 12, 11, 10, 9, 8, 7, 6, 5, or 4 weeks), such as no more than 8 weeks (e.g., the duration being 8, 7, 6, 5, or 4 weeks), wherein the treatment does not include administration of either interferon or ribavirin, and said at least 2 DAAs comprise Compound 2 (a pharmaceutically acceptable salt thereof) and sofosbuvir. Compound 2 (a pharmaceutically acceptable salt thereof) and sofosbuvir can be administered in therapeutically effective amounts to provide a SVR (for example, at least 75% SVR8, or preferably at least 80% SVR8, or highly preferably at least 90% SVR8, or most preferably at least 95% SVR8) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 11 weeks, or the duration being 10 weeks, or the duration being 9 weeks, or the duration being 8 weeks, or the duration being 7 weeks, or the duration being 6 weeks, or the duration being 5 weeks, or the duration being 4 weeks.

It will be understood that the specific dose level for any particular patient will depend upon a variety of factors including the activity of the specific compound employed, the age, body weight, general health, sex, diet, time of administration, route of administration, rate of excretion, drug combination, and the severity of the disease undergoing therapy.

In any method described herein wherein Compound 1 and Compound 2 are used, Compound 1 (or a pharmaceutically acceptable salt thereof) and Compound 2 (a pharmaceutically acceptable salt thereof) may be co-formulated in a single dosage form. Non-limiting examples of suitable dosage forms include liquid or solid dosage forms. Preferably, Compound 1 and Compound 2 are formulated in a single solid dosage form in which at least one of the DAAs is in an amorphous form, or highly preferably molecularly dispersed, in a matrix which comprises a pharmaceutically acceptable water-soluble polymer and a pharmaceutically acceptable surfactant. The other DAAs can also be in an amorphous form or molecularly dispersed in the matrix, or formulated in different form(s) (e.g., in a crystalline form). More preferably, each of the two DAAs is in an amorphous form, or highly preferably molecularly dispersed, in a matrix which comprises a pharmaceutically acceptable water-soluble polymer and a pharmaceutically acceptable surfactant.

In any method described herein wherein Compound 1, Compound 2 and sofosbuvir are used, Compound 1 (or a pharmaceutically acceptable salt thereof), Compound 2 (a pharmaceutically acceptable salt thereof) and sofosbuvir may be co-formulated in a single dosage form. Non-limiting examples of suitable dosage forms include liquid or solid dosage forms. Preferably, Compound 1, Compound 2 and sofosbuvir are formulated in a single solid dosage form in which at least one of the DAAs is in an amorphous form, or highly preferably molecularly dispersed, in a matrix which comprises a pharmaceutically acceptable water-soluble polymer and a pharmaceutically acceptable surfactant. The other DAAs can also be in an amorphous form or molecularly dispersed in the matrix, or formulated in different form(s) (e.g., in a crystalline form).

In any method described herein wherein Compound 2 and sofosbuvir are used, Compound 2 (or a pharmaceutically acceptable salt thereof) and sofosbuvir may be co-formulated in a single dosage form. Non-limiting examples of suitable dosage forms include liquid or solid dosage forms. Preferably, Compound 2 and sofosbuvir are formulated in a single solid dosage form in which at least one of the DAAs is in an amorphous form, or highly preferably molecularly dispersed, in a matrix which comprises a pharmaceutically acceptable water-soluble polymer and a pharmaceutically acceptable surfactant. The other DAAs can also be in an amorphous form or molecularly dispersed in the matrix, or formulated in different form(s) (e.g., in a crystalline form).

In any method described herein, the patient being treated can be a treatment-naïve patient.

In any method described herein, the patient being treated can be an interferon non-responder.

In any method described herein, the patient being treated can be an interferon null-responder.

In any method described herein, the patient being treated can be without cirrhosis.

In any method described herein, the patient being treated can be a cirrhotic patient.

In any method described herein, the patient being treated can be a patient with compensated cirrhosis.

In any method described herein where Compound 1 and Compound 2 are used, the DAAs employed in the method can consist of Compound 1 and Compound 2. In any method described herein where Compound 1 and Compound 2 are used, the DAAs employed in the method can consist of Compound 1 and Compound 2. In any method described herein where Compound 1 and Compound 2 are used, the DAAs employed in the method can consist of Compound 1 (or a pharmaceutically acceptable salt thereof) and Compound 2 (or a pharmaceutically acceptable salt thereof). In any method described herein where Compound 1 and Compound 2 are used, the DAAs employed in the method can consist of Compound 1 (or a pharmaceutically acceptable salt thereof), Compound 2 (or a pharmaceutically acceptable salt thereof) and a HCV nucleotide polymerase inhibitor. In any method described herein where Compound 1 and Compound 2 are used, the DAAs employed in the method can consist of Compound 1, Compound 2 and a HCV nucleotide polymerase inhibitor. In any method described herein where Compound 1 and Compound 2 are used, the DAAs employed in the method can consist of Compound 1 and Compound 2. In any method described herein where Compound 1 and Compound 2 are used, Compound 1 and Compound 2 can be administered with food. In any method described herein where Compound 1 and Compound 2 are used, Compound 1 and Compound 2 can be administered without food.

In any method described herein, the patient can have renal impairment, include hemodialysis. In any method described herein, the patient can have chronic kidney disease (CKD) stage 3b (eGFR 30 to < 45 mL/min/1.73 m²), stage 4 (eGFR 15 to < 30 mL/min/1.73 m²) or stage 5 (eGFR < 15 mL/min/1.73 m² or dialysis-dependent). In any method described herein, the patient can have HIV co-infection.

It is also contemplated a method of treating HCV, said method comprising administering to a patient in need thereof an effective amount of a combination of two or more DAAs. The treatment lasts 4 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 is (2R,6S,13aS,14aR,16aS,Z)-N-(cyclopropylsulfonyl)-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, and Compound 4 is as dimethyl (2S,2'S)-1,1'-((2S,2'S)-2,2'-(4,4'-((2S,5S)-1-(4-tert-butylphenyl)pyrrolidine-2,5,diyl)bis(4,1-phenylene))bis(azanediyl)bis(oxomethylene)bis(pyrrolidine-2,-diyl)bis(3 -methyl-1-oxobutane-2,1-diyl)dicarbamate, both of which are described in U.S. Patent Application Publication No. 2013/0102526, filed October 19, 2012 and entitled "Methods for Treating HCV", which is incorporated herein by reference in its entirety. Compound 3 preferably is co-administered with ritonavir. More preferably, Compound 3 is co-formulated with ritonavir. It is believed that the combination of Compound 3, Compound 4, and sofosbuvir, without ribavirin and interferon, can achieve at least about 80% SVR rate against HCV genotype 1 after 4-week treatment. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naive patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naive patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naïve patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naïve patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naive patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naive patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naive patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naive patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naive patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naive patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 5 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. It is believed that the combination of Compound 3, Compound 4, and sofosbuvir, without ribavirin and interferon, can achieve at least about 80% SVR rate against HCV genotype 1 after 5-week treatment. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naive patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naive patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 6 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naive patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 7 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naïve patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 8 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naive patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naive patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 9 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naive patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 10 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naïve patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naïve patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 11 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naive patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

It is further contemplated a method of treating HCV, said method comprising administering to a patient in need thereof an effective amount of a combination of two or more DAAs. The treatment lasts 12 weeks and does not include administration of any interferon or ribavirin (i.e., interferon- and ribavirin-free). The DAAs can be administered at the same or different dosing frequency. The patient being treated can be a treatment naive patient, a treatment experienced patient, including, but not limited to, a relapser, an interferon partial responder, an interferon non-responder (e.g., a null responder), or a patient unable to take interferon. The patient can be infected with, for example and without limitation, HCV genotype 1, such as HCV genotype 1a or HCV genotype 1b; or HCV genotype 2 or 3. The treatment according to this aspect can also be effective against other HCV genotypes. The DAAs can be administered around the same time or at different times, and can be co-formulated in a single formulation or formulated in different compositions. Each DAA can be selected from HCV protease inhibitors, HCV polymerase inhibitors, or HCV NS5A inhibitors. For instance, the combination of two or more DAAs can be a combination of at least one HCV protease inhibitor and at least one HCV polymerase inhibitor (e.g., a combination of at least one HCV protease inhibitor and at least one non-nucleoside polymerase inhibitor, or a combination of at least one HCV protease inhibitor and at least one nucleoside or nucleotide polymerase inhibitor, or a combination of at least one HCV protease inhibitor, at least one nucleoside or nucleotide polymerase inhibitor and at least one non-nucleoside inhibitor). For another instance, the combination of two or more DAAs can be a combination of at least one HCV protease inhibitor and at least one HCV NS5A inhibitor. For still another instance, the combination of two or more DAAs can be a combination of at least one HCV protease inhibitor, at least one HCV polymerase inhibitor, and at least one HCV NS5A inhibitor. For another instance, the combination of two or more DAAs can be a combination of at least two HCV polymerase inhibitors (e.g., a combination of at least two nucleoside or nucleotide polymerase inhibitors, or a combination of at least one nucleoside or nucleotide polymerase inhibitor and at least one non-nucleoside polymerase inhibitor, or a combination of at least two non-nucleoside polymerase inhibitors). For another instance, the combination of two or more DAAs can be a combination of at least two HCV protease inhibitors. For another instance, the combination of two or more DAAs can be a combination of at least two HCV NS5A inhibitors. For another instance, the combination of two or more DAAs can be a combination of at least one HCV polymerase inhibitor and at least one NS5A inhibitor (e.g., a combination of at least one HCV NS5A inhibitor and at least one non-nucleoside polymerase inhibitor, or a combination of at least one HCV NS5A inhibitor and at least one nucleoside or nucleotide polymerase inhibitor, or a combination of at least one HCV NS5A inhibitor, at least one nucleoside or nucleotide polymerase inhibitor and at least one non-nucleoside polymerase inhibitor). In one example, the combination of two or more DAAs is a combination of Compound 3, Compound 4, and sofosbuvir. Compound 3 preferably is co-administered with ritonavir. More preferably, Compound 3 is co-formulated with ritonavir. In another example, the combination of two or more DAAs is a combination of Compound 3, Compound 4, and sofosbuvir; and the patient is infected with HCV genotype 1. In another example, the combination of two or more DAAs is a combination of Compound 3, Compound 4, and sofosbuvir; and the patient is a treatment-naïve patient infected with HCV genotype 1. In another example, the combination of two or more DAAs is a combination of Compound 3, Compound 4, and sofosbuvir; and the patient is an interferon non-responder infected with HCV genotype 1. In another example, the combination of two or more DAAs is a combination of sofosbuvir, an HCV NS5A inhibitor, and another HCV polymerase inhibitor. In another example, the combination of two or more DAAs is a combination of sofosbuvir, an HCV NS5A inhibitor, and another HCV polymerase inhibitor; and the patient is infected with HCV genotype 1. In another example, the combination of two or more DAAs is a combination of sofosbuvir, an HCV NS5A inhibitor, and another HCV polymerase inhibitor; and the patient is a treatment-naive patient infected with HCV genotype 1. In another example, the combination of two or more DAAs is a combination of sofosbuvir, an HCV NS5A inhibitor, and another HCV polymerase inhibitor; and the patient is an interferon non-responder infected with HCV genotype 1. In yet another example, the combination of two or more DAAs comprises sofosbuvir, an HCV NS5A inhibitor, and an HCV protease inhibitor. In yet another example, the combination of two or more DAAs comprises sofosbuvir, an HCV NS5A inhibitor, and an HCV protease inhibitor; and the patient is infected with HCV genotype 1. In yet another example, the combination of two or more DAAs comprises sofosbuvir, an HCV NS5A inhibitor, and an HCV protease inhibitor; and the patient is a treatment-naïve patient infected with HCV genotype 1. In yet another example, the combination of two or more DAAs comprises sofosbuvir, an HCV NS5A inhibitor, and an HCV protease inhibitor; and the patient is an interferon non-responder infected with HCV genotype 1. In yet another example, the combination of two or more DAAs comprises sofosbuvir, an HCV protease inhibitor, and another HCV polymerase inhibitor. In yet another example, the combination of two or more DAAs comprises sofosbuvir, an HCV protease inhibitor, and another HCV polymerase inhibitor; and the patient is infected with HCV genotype 1. In yet another example, the combination of two or more DAAs comprises sofosbuvir, an HCV protease inhibitor, and another HCV polymerase inhibitor; and the patient is a treatment-naïve patient infected with HCV genotype 1. In yet another example, the combination of two or more DAAs comprises sofosbuvir, an HCV protease inhibitor, and another HCV polymerase inhibitor; and the patient is an interferon non-responder infected with HCV genotype 1. In another example, the combination of two or more DAAs is a combination of IDX21437, an HCV NS5A inhibitor, and another HCV polymerase inhibitor. In another example, the combination of two or more DAAs is a combination of IDX21437, an HCV NS5A inhibitor, and another HCV polymerase inhibitor; and the patient is infected with HCV genotype 1. In another example, the combination of two or more DAAs is a combination of IDX21437, an HCV NS5A inhibitor, and another HCV polymerase inhibitor; and the patient is a treatment-naive patient infected with HCV genotype 1. In another example, the combination of two or more DAAs is a combination of IDX21437, an HCV NS5A inhibitor, and another HCV polymerase inhibitor; and the patient is an interferon non-responder infected with HCV genotype 1. In yet another example, the combination of two or more DAAs comprises IDX21437, an HCV NS5A inhibitor, and an HCV protease inhibitor. In yet another example, the combination of two or more DAAs comprises IDX21437, an HCV NS5A inhibitor, and an HCV protease inhibitor; and the patient is infected with HCV genotype 1. In yet another example, the combination of two or more DAAs comprises IDX21437, an HCV NS5A inhibitor, and an HCV protease inhibitor; and the patient is a treatment-naïve patient infected with HCV genotype 1. In yet another example, the combination of two or more DAAs comprises IDX21437, an HCV NS5A inhibitor, and an HCV protease inhibitor; and the patient is an interferon non-responder infected with HCV genotype 1. In yet another example, the combination of two or more DAAs comprises IDX21437, an HCV protease inhibitor, and another HCV polymerase inhibitor. In yet another example, the combination of two or more DAAs comprises IDX21437, an HCV protease inhibitor, and another HCV polymerase inhibitor; and the patient is infected with HCV genotype 1. In yet another example, the combination of two or more DAAs comprises IDX21437, an HCV protease inhibitor, and another HCV polymerase inhibitor; and the patient is a treatment-naïve patient infected with HCV genotype 1. In yet another example, the combination of two or more DAAs comprises IDX21437, an HCV protease inhibitor, and another HCV polymerase inhibitor; and the patient is an interferon non-responder infected with HCV genotype 1. In yet another example, the combination of two or more DAAs comprises sofosbuvir, GS-5885, and another HCV polymerase inhibitor. In yet another example, the combination of two or more DAAs comprises sofosbuvir, GS-5885, and another HCV polymerase inhibitor; and the patient is infected with HCV genotype 1. In yet another example, the combination of two or more DAAs comprises sofosbuvir, GS-5885, and another HCV polymerase inhibitor; and the patient is a treatment-naïve patient infected with HCV genotype 1. In yet another example, the combination of two or more DAAs comprises sofosbuvir, GS-5885, and another HCV polymerase inhibitor; and the patient is an interferon non-responder infected with HCV genotype 1. In yet another example, the combination of two or more DAAs comprises sofosbuvir, GS-5816, and another HCV polymerase inhibitor. In yet another example, the combination of two or more DAAs comprises sofosbuvir, GS-5816, and another HCV polymerase inhibitor; and the patient is infected with HCV genotype 1. In yet another example, the combination of two or more DAAs comprises sofosbuvir, GS-5816, and another HCV polymerase inhibitor; and the patient is a treatment-naïve patient infected with HCV genotype 1. In yet another example, the combination of two or more DAAs comprises sofosbuvir, GS-5816, and another HCV polymerase inhibitor; and the patient is an interferon non-responder infected with HCV genotype 1. In yet another example, the combination of two or more DAAs comprises sofosbuvir, GS-5885, and an HCV protease inhibitor. In yet another example, the combination of two or more DAAs comprises sofosbuvir, GS-5885, and an HCV protease inhibitor; and the patient is infected with HCV genotype 1. In yet another example, the combination of two or more DAAs comprises sofosbuvir, GS-5885, and an HCV protease inhibitor; and the patient is a treatment-naïve patient infected with HCV genotype 1. In yet another example, the combination of two or more DAAs comprises sofosbuvir, GS-5885, and an HCV protease inhibitor; and the patient is an interferon non-responder infected with HCV genotype 1. In yet another example, the combination of two or more DAAs comprises sofosbuvir, GS-5816, and an HCV protease inhibitor. In yet another example, the combination of two or more DAAs comprises sofosbuvir, GS-5816, and an HCV protease inhibitor; and the patient is infected with HCV genotype 1. In yet another example, the combination of two or more DAAs comprises sofosbuvir, GS-5816, and an HCV protease inhibitor; and the patient is a treatment-naïve patient infected with HCV genotype 1. In yet another example, the combination of two or more DAAs comprises sofosbuvir, GS-5816, and an HCV protease inhibitor; and the patient is an interferon non-responder infected with HCV genotype 1. In yet another example, the combination of two or more DAAs comprises IDX21437, MK-8742, and another HCV polymerase inhibitor. In yet another example, the combination of two or more DAAs comprises IDX21437, MK-8742, and another HCV polymerase inhibitor; and the patient is infected with HCV genotype 1. In yet another example, the combination of two or more DAAs comprises IDX21437, MK-8742, and another HCV polymerase inhibitor; and the patient is a treatment-naïve patient infected with HCV genotype 1. In yet another example, the combination of two or more DAAs comprises IDX21437, MK-8742, and another HCV polymerase inhibitor; and the patient is an interferon non-responder infected with HCV genotype 1. In yet another example, the combination of two or more DAAs comprises IDX21437, MK-8742, and an HCV protease inhibitor. In yet another example, the combination of two or more DAAs comprises IDX21437, MK-8742, and an HCV protease inhibitor; and the patient is infected with HCV genotype 1. In yet another example, the combination of two or more DAAs comprises IDX21437, MK-8742, and an HCV protease inhibitor; and the patient is a treatment-naïve patient infected with HCV genotype 1. In yet another example, the combination of two or more DAAs comprises IDX21437, MK-8742, and an HCV protease inhibitor; and the patient is an interferon non-responder infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 100 or 200 mg Compound 3 together with 100 mg ritonavir once daily, 25 mg compound 4 once daily, and 400 mg sofosbuvir once daily; and the patient is an interferon non-responder infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is a treatment-naive patient infected with HCV genotype 1. In still another example, the combination of two or more DAAs is a combination of Compound 3, Compound 4, and sofosbuvir; and the method comprises administering 150 mg Compound 3 together with 100 mg ritonavir once daily, 25 mg compound 4 once daily, and 400 mg sofosbuvir once daily; and the patient is an interferon non-responder infected with HCV genotype 1.

In any method described herein, the HCV polymerase inhibitor recited therein can be IDX21437 (a uridine nucleotide analog HCV NS5B polymerase inhibitor, Idenix).

In any method described herein, the HCV polymerase inhibitor recited therein can also be IDX21459.

In any method described herein, the HCV NS5A inhibitor recited therein can be GS-5816.

In any method described herein, the HCV NS5A inhibitor recited therein can also be MK-8742.

In any method described herein, the patient being treated preferably is HCV genotype 1 patient.

It should be understood that the above-described embodiments and the following examples are given by way of illustration, not limitation. Various changes and modifications within the scope of the present invention will become apparent to those skilled in the art from the present description.

### Example 1. Clinical Modeling for Interferon-free DAA Combination Therapies

Treatment regimens comprising administration of Compound 1 and Compound 2 were evaluated using clinical models described in U.S. Patent Application Publication No. 2013/0102526, filed October 19, 2012 and entitled "Methods for Treating HCV", which is incorporated herein by reference in its entirety. These treatment regimens comprised administration of Compound 1 and Compound 2, but did not include administration of either interferon or ribavirin. Comparable SVR rates are expected for interferon-non responders.

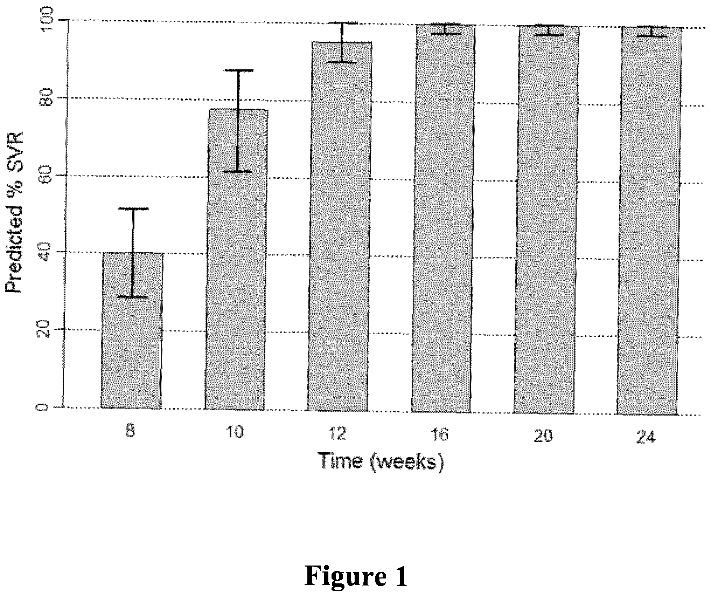

Figure 1 shows the predicted median SVR percentages and 90% SVR confidence intervals for 2-DAA regimens consisting of the use of Compound 1 (400 mg once daily) and Compound 2 (120 mg once daily) to treat genotype 1 naive subjects. Different treatment durations were assessed. The predicted SVR rate for a 12-week treatment was about 95%. As used in all of the figures of the present application, the vertical bar at the top of each SVR percentage column represents the 90% SVR confidence interval, and the x-axis ("Time (weeks)") indicates the duration of each treatment regimen.

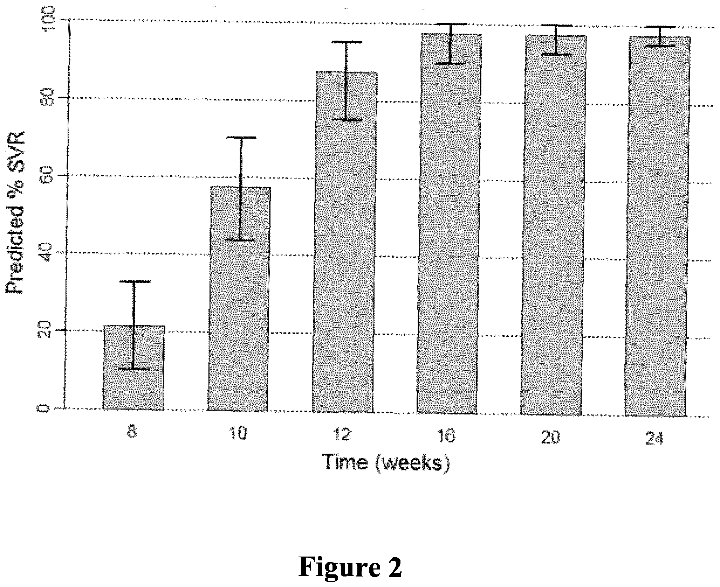

Figure 2 illustrates the predicted median SVR percentages and 90% SVR confidence intervals for 2-DAA regimens consisting of the use of Compound 1 (400 mg once daily) and Compound 2 (60 mg once daily) to treat genotype 1 naive subjects. Different treatment durations were assessed. The predicted SVR rate for a 12-week treatment was about 85-90%.

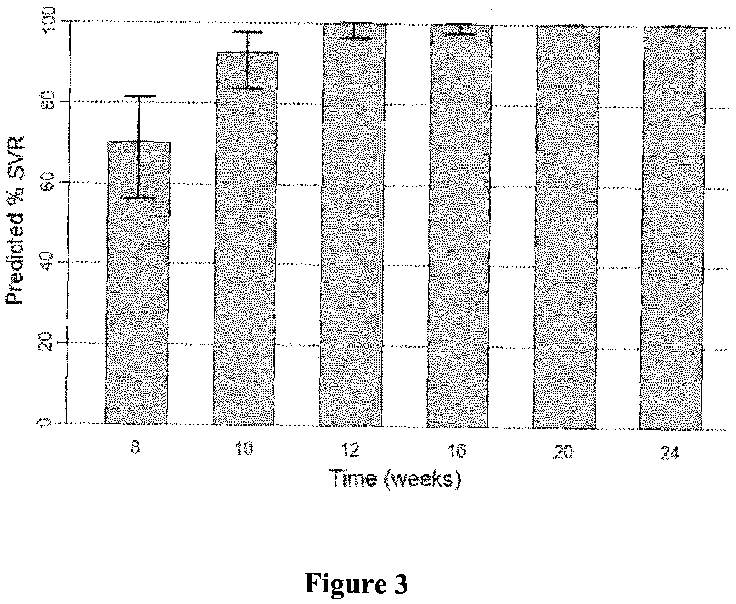

Figure 3 shows the predicted median SVR percentages and 90% SVR confidence intervals for 2-DAA regimens consisting of the use of Compound 1 (600 mg once daily) and Compound 2 (480 mg once daily) to treat genotype 1 naive subjects. Different treatment durations were assessed. The predicted SVR rate for a 12-week treatment was about 100%.

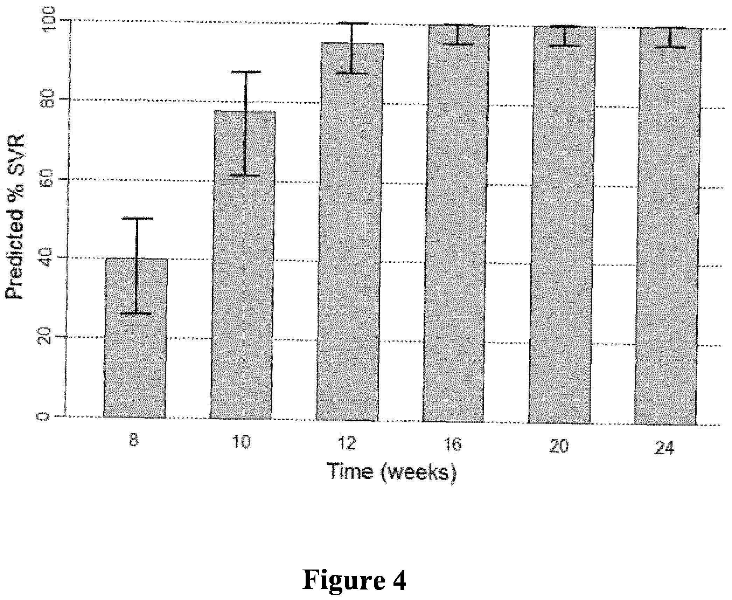

Figure 4 depicts the predicted median SVR percentages and 90% SVR confidence intervals for 2-DAA regimen consisting of the use of Compound 1 (400 mg once daily) and Compound 2 (120 mg once daily) to treat genotype 3 naive subjects. Different treatment durations were assessed. The predicted SVR rate for a 12-week treatment was about 95%.

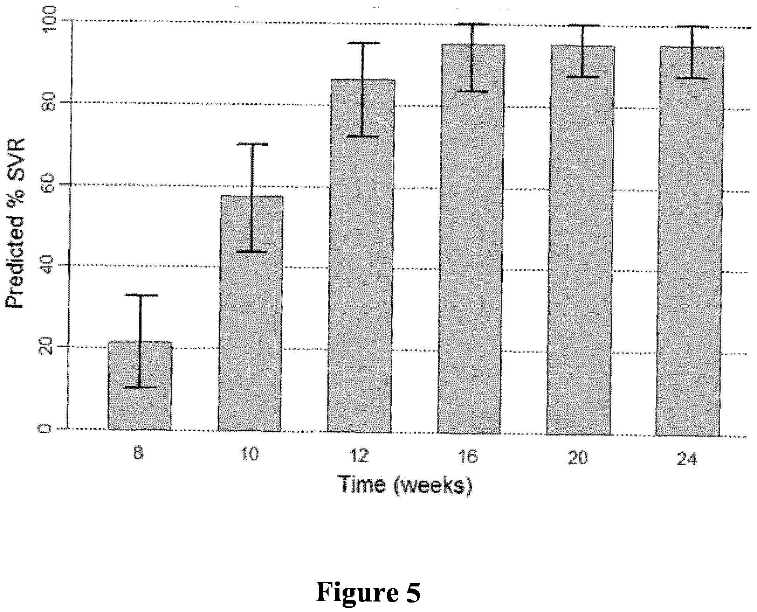

Figure 5 illustrates the predicted median SVR percentages and 90% SVR confidence intervals for 2-DAA regimen consisting of the use of Compound 1 (400 mg once daily) and Compound 2 (60 mg once daily) to treat genotype 3 naive subjects. Different treatment durations were assessed. The predicted SVR rate of a 12-week treatment was about 85-90%.

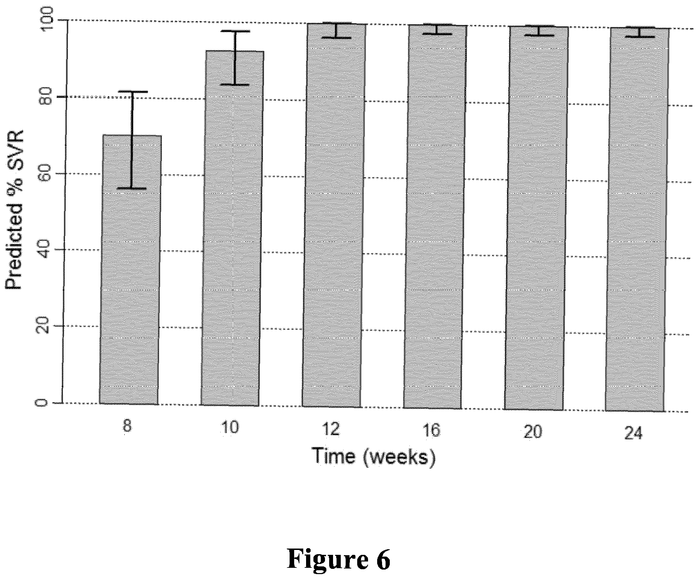

Figure 6 shows the predicted median SVR percentages and 90% SVR confidence intervals for 2-DAA regimens consisting of the use of Compound 1 (600 mg once daily) and Compound 2 (480 mg once daily) to treat genotype 3 naive subjects. Different treatment durations were assessed. The predicted SVR rate of a 12-week treatment was about 100%.

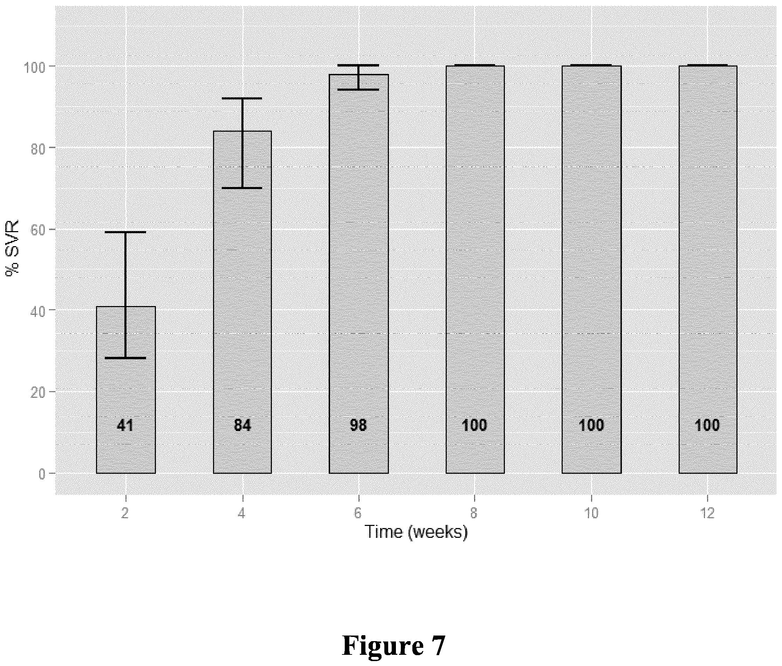

Treatment regimens comprising administration of Compound 1, Compound 2 and sofosbuvir, or Compound 2 and sofosbuvir, were also evaluated using the same clinical model. Figure 7 shows the predicted SVR for the treatment regimen consisting of the use of Compound 1 (400 mg once daily), Compound 2 (120 mg once daily) and sofosbuvir (400 mg once daily) to treat genotype 1 naive subjects. The treatment regimen did not include administration of either interferon or ribavirin. Different treatment durations were assessed. The predicted SVR rates of the 2-week, 4-week, 6-week, 8-week, 10-week, and 12-week treatment regimens were about 40%, 85%, 100%, 100%, 100%, and 100%, respectively. Comparable SVR rates are expected for interferon-non responders.

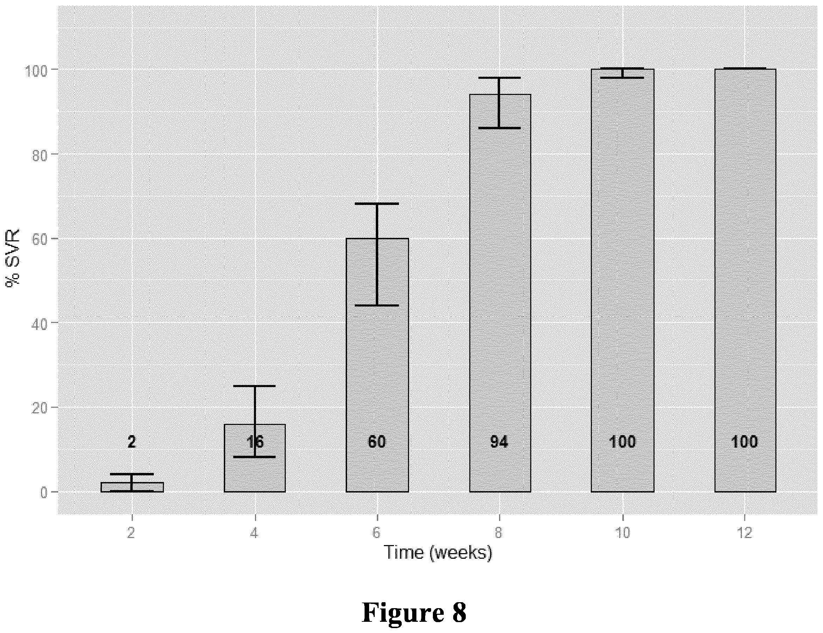

Figure 8 shows the predicted SVR for the treatment regimen consisting of the use of Compound 2 (120 mg once daily) and sofosbuvir (400 mg once daily) to treat genotype 1 naive subjects. The treatment regimen did not include administration of either interferon or ribavirin. Different treatment durations were assessed. The predicted SVR rates of the 6-week, 8-week, 10-week, and 12-week treatment regimens were about 60%, 95%, 100%, and 100%, respectively. Comparable SVR rates are expected for interferon-non responders.

### Example 2. Combination of Compound 1 and Compound 2 In Vitro

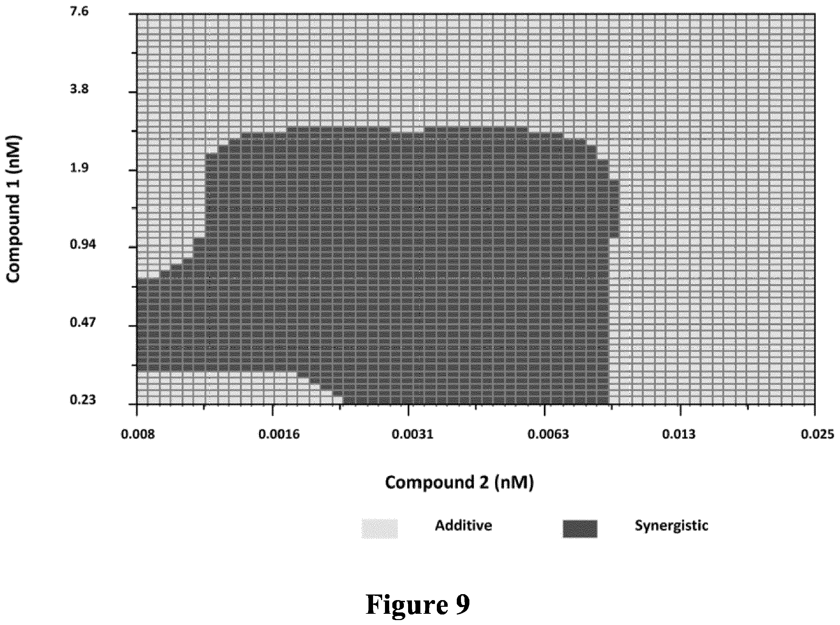

Figure 9 shows that the combination of Compound 1 and Compound 2 exhibits significant synergistic effect on HCV inhibition as tested in HCV GT 1b Con-1 replication cells. The result was generated using Prichard and Shipman model (Prichard et al. ANTIVIRAL RESEARCH 14:181-205 (1990)).

Compound 1 inhibited replication of HCV stable subgenomic replicons containing NS3 genes from GT 1a, 1b, 2a, 3a, 4a, or 6a with EC₅₀ values ranging from 0.85 to 2.8 nM. Of note, Compound 1 was potent against replicon containing GT3a protease, with an EC₅₀ value of 1.6 nM. Compound 1 retained its activity against common GTla and 1b variants at NS3 amino acid positions 155 and 168 that conferred resistance to other HCV protease inhibitors (Pis). Resistant colony selection studies in GTla and 1b subgenomic replicon cells identified A156T in GTla and A156V in GT1b as the most frequent variants, which conferred 1400- and 1800-fold reduced susceptibility to Compound 1, respectively. However, these variants had *in vitro* replication capacities of only 1.5% and 9.2% that of their corresponding wild-type replicons. In a replicon containing GT3a NS3 protease, Compound 1 selected very few colonies at concentrations ≥ 100-fold over its EC50 value. The colonies that survived the selection contained either A156G alone, or Q168R co-selected with Y56H, which conferred 1500- or 1100-fold loss in susceptibility to Compound 1, respectively.

**Table 2. Antiviral Activity of Compound 1 in the HCV Subgenomic Stable Replicon Cell Culture Assay**

| **HCV Replicon Subtype** | **N**^{b} | **0% Human Plasma**^{a} **Mean EC₅₀, nM, ± Std. Dev.** |
|---|---|---|
| Genotype 1a | 9 | 0.85 ± 0.15 |
| Genotype 1b | 8 | 0.94 ± 0.35 |
| Genotype 2a | 2 | 2.7 ± 1.1 |
| Genotype 3a | 2 | 1.6 ± 0.49 |
| Genotype 4a | 4 | 2.8 ± 0.41 |
| Genotype 6a | 4 | 0.86 ± 0.11 |

| | | |
|---|---|---|
| a. The 0% human plasma assay contains 5% fetal bovine serum b. Number of independent replicates | | |

**Table 3. Antiviral Activity of Compound 1 in the HCV Subgenomic Stable Replicon Cell Culture Assay**

| **HCV Replicon Subtype** | **N**^{b} | **40% Human Plasma^{a} Mean EC₅₀, nM, ± Std. Dev.** |
|---|---|---|
| Genotype 1a | 10 | 5.3 ± 1.0 |
| Genotype 1b | 8 | 10 ± 5.0 |

| | | |
|---|---|---|
| a. The 0% human plasma assay contains 5% fetal bovine serum b. Number of independent replicates | | |

When tested against common HCV genotype 1 NS3 resistance-associated variants, such as V36M, R155K, D168A and D168V in GT 1a (H77), or T54A, R155K, D168V and V170A in GT 1b (Con-1), Compound 1 showed inhibitory activity nearly equivalent to that against wild-type HCV replicon. Compound 1 was also shown to have potent activity against many NS5A inhibitor and NS5B inhibitor resistance-associated variants *in vitro* (e.g., M28T, M28V, Q30D, Q30R, Y93C, Y93H, Y93N, L31V+Y93H, C316Y, M414T, Y448C, Y448H, S556G and S559G in GT 1a, and L28T, Y93H, S282T, C316Y, Y448H and S556G in GT 1b).

### Example 3. High SVR in HCV Genotype 1 (GT1) Non-Cirrhotic Treatment-Naive Patients or Pegylated Interferon/Ribavirin Null Responders Treated with the Combination of Compound 1 and Compound 2

Compound 1 and Compound 2 are characterized by potent pangenotypic in vitro antiviral activity against major HCV genotypes (GTs), including activity against key known resistance-associated variants and a high barrier to resistance selection. Monotherapy with Compound 1 or Compound 2 resulted in a mean 4 log₁₀ IU/mL decline from baseline in HCV plasma viral load in GT1-infected subjects with and without compensated cirrhosis.

In this phase 2 study, treatment with Compound 1 and Compound 2 for 12 weeks is evaluated in HCV GT1-infected subjects without cirrhosis. Non-cirrhotic GT1-infected treatment-naïve (TN) or pegylated interferon/ribavirin (pegIFN/RBV) null responder subjects received once-daily Compound 1 200 mg + Compound 2 120 or 40 mg for 12 weeks, and subsequently were followed for 24 weeks. Efficacy was measured by sustained virologic response after the last dose of study drug (SVR). Safety was evaluated by adverse event (AE) monitoring, laboratory testing, and other standard assessments.

79 subjects (male, 52%; median [range] age, 54.0 [26.0-70.0] years; GT1a, 81%; GT1b, 19%; TN, 63%; pegIFN/RBV null responders, 37%; fibrosis >F2, 25%; median [range] HCV RNA log₁₀ IU/mL, 6.8 [4.4-7.5]) were enrolled, 40 received Compound 1 200 mg + Compound 2 120 mg, and 39 received Compound 1 200 mg and Compound 2 40 mg. SVR 4 weeks after the last dose of study drug (SVR4) was achieved in 29 of 29 (100%) pegIFN/RBV null responders and 49 of 50 (98%) TN subjects. There were no treatment-related serious AEs or clinically relevant laboratory findings. The most common AEs (reported in >5% of subjects) were fatigue, headache, nausea, diarrhea, and anxiety.

Once-daily 12-week treatment with the combination of Compound 1 and Compound 2 of GT1 infection in non-cirrhotic TN and pegIFN/RBV null responders resulted in high SVR4 (98%-100%) rates. One treatment relapse was observed.

Non-cirrhotic HCV GT1-infected patients treated with the combination of Compound 1 and Compound 2 for 12 weeks achieved high SVR12 rates, regardless of prior treatment experience or presence of baseline variants.

Treatment with the combination of Compound 1 and Compound 2 for 12 weeks is also expected to achieve high SVR in GT1 subjects with compensated cirrhosis. Likewise, high SVR is expected in GT1 patients if treated with the combination of Compound 1 and Compound 2 once-daily for only 8 weeks. Suitable dosing includes, but is not limited to, Compound 1 300 mg + Compound 2 120 mg once daily, or Compound 1 200 mg + Compound 2 80 mg once daily.

### Example 4. High SVR Achieved with Compound 1 and Compound 2 in Non-Cirrhotic Treatment-Naïve and Treatment-Experienced Patients with HCV Genotype 2 (GT2) Infection

As shown in Example 3, Compound 1 and Compound 2 are potent inhibitors against GT1. Compound 1 and Compound 2 have comparable in vitro antiviral potency against GT2. This Example evaluates the efficacy and safety of Compound 1 and Compound 2 with or without ribavirin (RBV) in non-cirrhotic GT2-infected treatment-naïve (TN) and pegylated interferon/RBV (pegIFN/RBV) treatment experienced (TE) subjects.

Subjects received 12 weeks of Compound 1 300 mg + Compound 2 120 mg (Arm A), Compound 1 200 mg + Compound 2 120 mg (Arm B), or Compound 1 200 mg + Compound 2 120 mg + RBV (Arm C). DAAs were dosed once daily; weight-based RBV (1000 or 1200 mg) was dosed twice daily. Subjects were then followed for 24 weeks. Efficacy was measured by sustained virologic response after the last dose of study drug (SVR). Safety was evaluated by monitoring adverse events (AEs), laboratory tests, and vital signs.

75 subjects were treated in Arms A-C (n=25 each); 74 had GT2, and 1 subject initially randomized to Arm B was determined to have GT3a infection. Subjects were male, 63%; median (range) age, 57.0 (20.0-69.0) years; GT2b, 81%; TN, 88%; TE, 12%; F0-F2, 87%; F3, 13%; median (range) baseline HCV RNA log₁₀ IU/mL, 7.1 (4.7-7.8). No subjects have experienced virologic failure. One subject in Arm A prematurely discontinued study drugs and was lost to follow-up. The SVR4 rates (ITT analysis) are 96% (24/25), 100% (24/24), and 100% (25/25) in Arms A, B, and C, respectively. Most AEs were mild, with the most common DAA-related AEs being fatigue, nausea, headache, and diarrhea. There were no serious DAA-related AEs. Typical reductions in hemoglobin were observed in the RBV-containing arm.

Compound 1+Compound 2 with or without RBV for 12 weeks was highly effective and well tolerated, achieving SVR4 rates of 96%-100%.

The once daily regimen of the combination of Compound 1 and Compound 2 was well tolerated and demonstrated high SVR12 rates, regardless of prior treatment experience or presence of baseline variants in non-cirrhotic patients with GT2 infection.

Treatment with the combination of Compound 1 and Compound 2 for 12 weeks is also expected to achieve high SVR in GT2 subjects with compensated cirrhosis. Likewise, high SVR is expected in GT2 patients if treated with the combination of Compound 1 and Compound 2 once-daily for only 8 weeks. Suitable dosing includes, but is not limited to, Compound 1 300 mg + Compound 2 120 mg once daily, or Compound 1 200 mg + Compound 2 80 mg once daily.

### Example 5. High SVR Achieved with Compound 1 and Compound 2 in Non-Cirrhotic Treatment-Naïve and Treatment-Experienced Patients with HCV Genotype 3 (GT3) Infection

As shown in Example 3, Compound 1 and Compound 2 are potent inhibitors against GT1. Compound 1 and Compound 2 have comparable in vitro antiviral potency against GT3. This Example evaluates the efficacy and safety of Compound 1 and Compound 2 with or without ribavirin (RBV) in non-cirrhotic GT3-infected treatment-naïve (TN) and pegylated interferon/RBV (pegIFN/RBV) treatment experienced (TE) subjects.

Subjects received 12 weeks of Compound 1 300 mg + Compound 2 120 mg (Arm D), Compound 1 200 mg + Compound 2 120 mg (Arm E), Compound 1 200 mg + Compound 2 120 mg + RBV (Arm F), or Compound 1 200 mg + Compound 2 40 mg (Arm G). DAAs were dosed once daily; weight-based RBV (1000 or 1200 mg) was dosed twice daily. Efficacy was measured by sustained virologic response after the last dose of study drug (SVR). Safety was evaluated by monitoring adverse events (AEs), laboratory tests, and vital signs.

120 GT3-infected subjects were treated in Arms D (n=30), E (n=29), F (n=31), or G (n=30). Subjects were male, 56%; median age, 52.0 years; GT3a, 98%; TN, 92%; TE, 8%; fibrosis >F2, 15%; median baseline HCV RNA log₁₀ IU/mL, 6.7. There has been 1 virologic failure in each treatment arm (n=4), 3 of which were in pegIFN/RBV TE subjects. One subject in Arm G was lost to follow-up at the week 2 visit. The SVR4 rate was 96% (27/28), 96% (27/28), 97% (29/30) and 93% (27/29) in Arms D, E, F, and G, respectively. AEs were mostly mild, with most common DAA-related AEs being fatigue, nausea, and headache. There were no serious DAA-related AEs; 1 subject discontinued due to DAA- and RBV-related AEs of abdominal pain and heat sensation. Typical reductions in hemoglobin were observed in the RBV containing arm (Arm F).

Compound 1+Compound 2 treatment for 12 weeks with or without RBV in TN or TE non-cirrhotic HCV GT3-infected subjects was well tolerated. Promising SVR4 rates of 93%-96% were achieved without RBV. Further testing showed that Arms D, E, F and G achieved 93%, 93%, 94% and 83% SVR12, respectively.

Treatment with the combination of Compound 1 and Compound 2 for 12 weeks is also expected to achieve high SVR in GT3 subjects with cirrhosis. Likewise, high SVR is expected in GT3 patients if treated with the combination of Compound 1 and Compound 2 once-daily for only 8 weeks. Suitable dosing includes, but is not limited to, Compound 1 300 mg + Compound 2 120 mg once daily, or Compound 1 200 mg + Compound 2 80 mg once daily.

### Example 6. Drug-Drug Interactions between Compound 1 and Compound 2 with Cyclosporine or Tacrolimus in Healthy Subjects

Compound 1 + Compound 2 combination demonstrated high sustained virologic response rate in Phase 2 studies. Two Phase 1, open-label studies were conducted to assess the pharmacokinetics, safety and tolerability when Compound 1 + Compound 2 is coadministered with immunosuppressants cyclosporine or tacrolimus.

Healthy adults subjects received single doses of cyclosporine 100 mg (n=12) or tacrolimus 1 mg (n=12) alone or in combination with Compound 1 300 mg QD (i.e., once daily) + Compound 2 120 mg QD. Intensive blood sampling for determination of cyclosporine, tacrolimus, Compound 1 and Compound 2 concentrations was performed and pharmacokinetic parameters (maximum observed concentration [Cₘₐₓ], area under the concentration-time curve [AUCₜ or AUC_{inf}] and trough concentration [C₂₄]) were estimated. Safety and tolerability were assessed throughout the study.

Cyclosporine Cₘₐₓ, AUCₜ, and AUC_{inf} in blood were minimally affected (≤14% change) when coadministered with steady-state Compound 1 + Compound 2. Steady-state Cₘₐₓ, AUC₂₄, and C₂₄ in plasma were slightly increased for Compound 1 (30%, 37%, and 34%, respectively) and for Compound 2 (11%, 22%, and 26%, respectively) when coadministered with cyclosporine. Tacrolimus Cₘₐₓ, AUCₜ, and AUC_{inf} in blood were slightly increased (50%, 53%, and 45%, respectively) when coadministered with steady-state Compound 1 + Compound 2. Steady-state Cₘₐₓ, AUC₂₄, and C₂₄ in plasma were minimally affected for Compound 1 (≤11% change) and for Compound 2 (≤2% change) when coadministered with tacrolimus.

No serious adverse events were observed in either study. There were no patterns to the adverse events reported, and no new safety issues were identified.

No dose adjustment should be required for Compound 1, Compound 2, and cyclosporine when coadministered. No dose adjustment should be required for Compound 1 and Compound 2 when coadministered with tacrolimus. It may be considered that subjects receiving tacrolimus should continue to use their current dose when initiating treatment with DAAs, and reduce the dose of tacrolimus if necessary based on therapeutic monitoring.

### Example 7. Absence of Significant Drug-Drug Interactions between Compound 1/Compound 2 and Methadone or Buprenorphine/Naloxone in Subjects on Opioid Maintenance Therapy

A Phase 1, open-label study was conducted to assess the pharmacokinetics, safety and tolerability of Compound 1 + Compound 2 and methadone or buprenorphine/naloxone. Otherwise healthy adults subjects on individualized regimens of methadone (n=12) or buprenorphine/naloxone (n=12) for opioid addiction received Compound 1 300 mg QD + Compound 2 120 mg QD for 7 days. Intensive blood sampling for determination of methadone, buprenorphine, norbuprenorphine, naloxone, Compound 1 and Compound 2 concentrations was performed and pharmacokinetic parameters (maximum observed concentration [Cₘₐₓ], area under the concentration-time curve [AUC₂₄ or AUCₜ] and trough concentration [C₂₄]) were estimated. Safety and tolerability were assessed throughout the study. Potential opioid withdrawal or overdose symptoms (pharmacodynamics) were assessed with validated instruments including the short opiate withdrawal scale, desire for drugs questionnaire, and pupillometry measurements throughout the study.

For subjects on methadone maintenance therapy, dose-normalized Cₘₐₓ, AUC₂₄, and C₂₄ for Rand S-methadone were unaffected by coadministration with Compound 1 and Compound 2 at steady-state (≤5 % change). For subjects on buprenorphine/naloxone maintenance therapy, dose-normalized Cₘₐₓ, AUC₂₄, and C₂₄ were slightly increased for buprenorphine (8%, 17%, and 24%, respectively) and norbuprenorphine (25%, 30%, and 21%, respectively) when coadministered with Compound 1 and Compound 2 at steady-state; naloxone dose-normalized Cₘₐₓ and AUCt were minimally affected (≤12% change). Pharmacodynamics of the methadone or buprenorphine/naloxone regimens were not significantly impacted by coadministration with Compound 1 or Compound 2 for either regimen. Compound 1 and Compound 2 exposures following coadministration with methadone or buprenorphine/naloxone were similar to the observed values in previous studies.

Subjects experienced adverse events of mild intensity, with the most common (reported in >5 subjects) being abdominal pain, constipation, and headache; all subjects completed the study. There were no clinically relevant abnormal laboratory abnormalities, ECG or vital sign findings.

No dose adjustments should be required for coadministration of Compound 1 and Compound 2 with methadone or buprenorphine/naloxone. No pharmacodynamic interaction is expected.

### Example 8. Pharmacokinetics of Coadministration of Pan-Genotypic, Direct Acting Antiviral Agents, Compound 1 and Compound 2, with or without Ribavirin for 12 weeks in HCV Infected Subjects without Cirrhosis

Pharmacokinetics of Compound 1 and Compound 2 with or without ribavirin (RBV) were evaluated. Two open-label, multicenter studies were conducted to evaluate the efficacy, safety, and pharmacokinetics of co-administration of Compound 1 (200 or 300 mg QD) and Compound 2 (40 or 120 mg QD) with or without RBV in GT1-, GT2- or GT3-infected subjects. Blood samples for pharmacokinetic analysis were collected throughout the study treatment period. Compound 1 and Compound 2 pharmacokinetics following a single dose (Day 1) and at steady state (Week 4) were assessed by non-compartmental methods.

A total of 274 subjects received Compound 1 and Compound 2 with or without RBV. Both Compound 1 and Compound 2 showed rapid absorption with Tmax ranging from 2 to 4 hours. Steady state Compound 1 exposure (area under the curve from 0 to 4 hour) following 300 mg was 2570 ng.h/mL, approximately 3.7-fold of the exposure following 200 mg administration. Coadministration of either 40 mg or 120 mg Compound 2 each with 200 mg Compound 1 resulted in Compound 2 exposure of 157 or 372 ng.h/mL, respectively. Compound 1 300 mg increased 120 mg Compound 2 exposure by an additional 20% to 30%. Minimal accumulation in Compound 1 or Compound 2 exposure was observed at Week 4 compared to Day 1. Compound 2 had minimal impact on Compound 1 exposures, however, Compound 1 200 mg and 300 mg increased 120 mg Compound 2 exposures to 3- to 4-fold of Compound 2 exposures when administered alone. HCV genotype and RBV coadministration had no impact on Compound 1 or Compound 2 exposures.

Compound 1 exhibited non-linear pharmacokinetics with more than dose-proportional increase in exposures with increasing doses, while Compound 2 exposures increased in an approximately dose-proportional manner when coadministered with Compound 1. Compound 2 had minimal impact on Compound 1, while Compound 1 increased Compound 2 exposures, with the increase in Compound 2 exposure being dependent on Compound 1 dose. Compound 1 or Compound 2 had minimal accumulation in exposures following multiple dosing in HCV-infected subjects.

### Example 9. Drug-drug interactions between Compound1/Compound 2 and sofosbuvir

A Phase 1 study was conducted to evaluate any potential interactions during co-administration of Compound 1 + Compound 2 with sofosbuvir. This was an open label, randomized, multiple-dose, non-fasting study in 16 healthy subjects who were assigned 1:1 to one of two cohorts to receive either Compound 1 400 mg QD + Compound 2 120 mg QD or sofosbuvir 400 mg QD for 7 days (Period 1), followed by the combination of Compound 1 400 mg QD + Compound 2 120 mg QD with sofosbuvir 400 mg QD for 7 additional days (Period 2). Intensive pharmacokinetic assessments were performed on Study Days 1 and 7 in each period. Pharmacokinetic interaction between Compound 1 + Compound 2 and sofosbuvir were assessed by a repeated-measures analysis using SAS. Safety was evaluated through assessment of adverse events, vital signs, ECGs and clinical laboratory tests.

Coadministration with steady-state Compound 1 and Compound 2 increased sofosbuvir Cₘₐₓ and AUC₂₄ by 66% and 125%, respectively; Cₘₐₓ and AUC₂₄ for the major circulating sofosbuvir metabolite GS-331007 were minimally affected (≤ 21% difference) and C₂₄ was increased by 85%. Compound 1 and Compound 2 exposures were minimally affected by sofosbuvir (≤16% difference). There were no patterns to the adverse events reported, and no new safety issues were identified.

This study showed that no dose adjustments are needed for coadministration of Compound 1 and Compound 2 with sofosbuvir.

### Example 10. Pharmacokinetics, Tolerability and Safety of Compound 2 following Single and Multiple Doses in Healthy Subjects

The study's objectives were to determine the pharmacokinetics (PK), safety, and tolerability of Compound 2 following single ascending doses (SAD) and multiple ascending doses (MAD) and effect of food on Compound 2 PK in healthy adults. This was a blinded, randomized, placebo-controlled Phase 1 study. Seven Compound 2 doses ranging from 1.5 mg to 600 mg were evaluated in the SAD portion (n=53, 3:1 active to placebo ratio). Compound 2 doses of 30 mg to 600 mg QD were evaluated in MAD portion for 10 days (n=39, 4:1 active to placebo ratio). The effect of food on Compound 2 120 mg was assessed in a crossover fashion in 12 healthy subjects. The PK parameters of Compound 2 were estimated using non-compartmental methods. Safety and tolerability were assessed throughout the study.

Compound 2 exposures increased in a greater than dose proportional manner across the 1.5 mg to 120 mg dose range, while PK was linear across the 120 mg to 600 mg dose range. Compound 2 plasma concentration reached Tₘₐₓ at 3 to 5 hours. Following Compound 2 QD dosing for 10 days, the Compound 2 steady state exposures were 53% higher compared to exposures after the first dose. Compound 2 half-lives ranged from 20 to 22 hours. Steady state of Compound 2 was attained by Study Day 5. Food had minimal effect on the bioavailability of Compound 2 (<14%). All adverse events were assessed as mild. No clinically significant vital signs or laboratory measurements were observed during the course of the study.

This study showed that Compound 2 PK support QD dosing and administration without regard to food. All dose levels were well tolerated and a maximum tolerated dose was not reached in the SAD and MAD portions of the study.

### Example 11. High SVR in HCV Genotype 1 Non-Cirrhotic Treatment-Naive or -Experienced Patients with the Combination of Compound 1 and Compound 2 for 8 Weeks

Compound 1 and Compound 2 co-administered for 12 weeks showed high sustained virologic response (SVR) rates and was well tolerated in non-cirrhotic patients with HCV genotype 1 (GT1) infection. This Example shows the efficacy and safety data of the combination of Compound 1 and Compound 2 administered for 8 weeks in non-cirrhotic patients with GT1 infection.

Treatment-naive or pegylated interferon treatment-experienced patients received once-daily Compound 1 300 mg + Compound 2 120 mg for 8 weeks. SVR at post-treatment week 4 (SVR₄; HCV RNA measured using COBAS TaqMan® RT-PCR [lower limit of detection of 15 IU/mL and lower limit of quantitation of 25 IU/mL]) and safety data were determined.

34 patients were enrolled: 56% male, 97% white, 71% GT1a, 68% non-CC IL28B, 15% had an F3 fibrosis stage at baseline, and 15% were treatment experienced. The median (range) HCV RNA log₁₀ IU/mL was 6.5 (2.9-7.5) at baseline, and 38% of patients had HCV RNA ≥ 6,000,000 IU/mL. After 8-week treatment, all 34 (100%) patients achieved SVR₄ and 97% of the patients achieved SVR12. One patient did not achieve SVR12 after achieving SVR4 due to death from advanced cancer not related to study drugs. There were no additional serious or severe AEs reported. The most frequent AEs observed in >10% of patients were fatigue (21%) and diarrhea (12%).

This study showed that the combination of Compound 1 and Compound 2 was well tolerated and achieved high SVR rate in all treatment-naïve or -experienced patients with GT1 infection who completed 8 weeks of treatment, regardless of baseline viral load, baseline viral load, prior treatment history, or presence of baseline NS3 and/or NS5A variants.

### Example 12. High SVR Rates With the Combination of Compound 1 + Compound 2 for 8 Weeks in Non-Cirrhotic Patients with HCV Genotype 2 Infection

Compound 1 + Compound 2 for 12 weeks was well tolerated and achieved sustained virologic response (SVR) rates between 97 - 100% in non-cirrhotic patients with HCV genotype (GT) 1 or 2 infection. In this Example, Compound 1 + Compound 2 was co-administered to HCV GT2 patients for a shorter duration of 8 weeks.

Non-cirrhotic treatment-naive patients or pegylated interferon/ribavirin treatment-experienced non-responders received once-daily Compound 1 300 mg + Compound 2 120 mg for 8 weeks. HCV RNA <25 IU/mL at post-treatment weeks 4 (SVR4) and safety were evaluated.

54 patients with GT2 infection (70% GT2b; 59% non-CC IL28B genotype; 13% treatment-experienced) were enrolled, respectively. Mean baseline HCV RNA log₁₀ IU/mL ± standard deviation was 6.6 ± 0.8 for these GT2-infected patients, with 57% of patients who had baseline levels ≥6M IU/mL . SVR4 was achieved by 98% (53/54) of GT2-infected patients. The GT2-infected patient without SVR4 was lost to follow up after week 6, where HCV RNA was not detected. There were no other discontinuations due to AEs. AEs were mostly mild (Grade 1), with the most common AEs being fatigue and headache.

This study showed that the combination of Compound 1 and Compound 2 administered for 8 weeks in non-cirrhotic patients with HCV GT2 infection was well tolerated and achieved high SVR, regardless of baseline viral load or prior treatment history.

### Example 13. High SVR Rates With Compound 1 + Compound 2 Co-Administered for 8 Weeks in Non-Cirrhotic Patients with HCV Genotype 3 Infection

Treatment-naïve HCV GT3-infected patients without cirrhosis received once-daily Compound 1 300 mg + Compound 2 120 mg for 8 weeks. SVR4 (HCV RNA below the lower limit of quantitation [25 IU/mL] at post-treatment week 4) and safety were assessed.

29 patients were enrolled: 52% male, 90% white, 86% GT3a, and 62% non-CC IL28B. The median (range) HCV RNA log₁₀ IU/mL was 6.3 (5.0 - 7.5) and 24% of patients had HCV RNA≥6M IU/mL at baseline. SVR4 was achieved by 97% (28/29) of patients. No patients experienced virologic failure to date. One patient discontinued the study after treatment week 6 (HCV RNA undetectable at this visit) due to intolerance of blood draws. No patients discontinued due to adverse events (AEs) or experienced serious AEs. The majority of AEs were mild in severity, with the most common AEs (>10% of patients) reported for patients being headache and fatigue.

This study showed that the combination of Compound 1 and Compound 2, co-administered for 8 weeks in treatment-naive, non-cirrhotic patients with HCV GT3 infection was well-tolerated and achieved high SVR rates.

### Example 14. Antiviral Activity of Compound 2 In Combination With Paritaprevir/Ritonavir and Ribavirin Against Hepatitis C Virus Genotype 3 Infection

Efficacy, pharmacokinetics, and safety of Compound 2 co-administered with paritaprevir/ritonavir and ribavirin were evaluated in this phase 2, open-label, multicenter study in treatment-naïve non-cirrhotic patients with HCV genotype 3 infection. Ten patients, all genotype 3a, received 120 mg Compound 2 and 150/100 mg paritaprevir/ritonavir once daily with weight-based ribavirin for 12 weeks. A total daily dose of 1000 mg ribavirin if the patient's body weight was <75 kg or 1200 mg if body weight was ≥75 kg was divided twice daily (BID).

Nine (90%) patients achieved sustained virologic response at post-treatment weeks 12 and 24. One patient experienced virologic failure at treatment week 6. Sequence analyses for HCV variants in samples from this patient identified A166S in NS3 at baseline and after breakthrough, as well as A30K at baseline and linked S24F+M28K+A30K variants in NS5A after breakthrough. Neither NS3 A166S nor NS5A A30K variant confers any resistance to paritaprevir or Compound 2, respectively. However, NS5A S24F+M28K+A30K linked variant confers a >5000-fold increase in Compound 2 EC₅₀ relative to that of the wild-type replicon. This patient's Compound 2 exposure was comparable to the cohort, while paritaprevir and ritonavir exposures were the lowest of all patients. No serious or severe adverse events and adverse events leading to early discontinuation were reported.

The study confirmed that Compound 2 in combination with paritaprevir/ritonavir and ribavirin is effective against HCV genotype 3 infection.

### Example 15. 100% SVR4 and Favorable Safety of Compound 1 + Compound 2 Administered for 12 Weeks in Non-Cirrhotic Patients with Genotypes 4, 5, or 6 Infection

This study evaluated the efficacy and safety of Compound 1 and Compound 2 co-administered for 12 weeks in non-cirrhotic patients with HCV genotypes 4, 5, or 6 infection. Treatment-naive or pegylated interferon/ribavirin treatment-experienced patients received once-daily Compound 1 300 mg + Compound 2 120 mg for 12 weeks. Sustained virologic response at post-treatment week 4 (SVR4; HCV RNA measured using COBAS TaqMan® RT-PCR [lower limit of detection of 15 IU/mL and lower limit of quantitation of 25 IU/mL]) and safety data was assessed.

A total of 34 patients with genotype 4 (n=22; 65%), 5 (n=1; 3%), or 6 (n=11; 32%) infection were enrolled: 53% male, 59% white, 62% had non-CC IL28B, and 15% were treatment-experienced. The median (range) HCV RNA log₁₀ IU/mL was 6.4 (4.6 - 7.4) at baseline, and 35% of patients had HCV RNA ≥ 6,000,000 IU/mL. SVR4 was achieved by all 34 (100%) patients with genotypes 4, 5, or 6 infection. Adverse events (AEs) reported were deemed mostly Grade 1 (mild) in severity, with common AEs in >5% of all patients being headache (24%), diarrhea (15%), fatigue (12%), nausea (9%), arthralgia (6%), dizziness (6%), dry mouth (6%), and flatulence (6%). No severe AEs, serious AEs, premature discontinuations due to AEs were reported. While receiving therapy, no liver function or other laboratory abnormalities were observed.

This study showed that the combination of Compound 1 and Compound 2 was well tolerated and demonstrated 100% SVR4 in non-cirrhotic patients with genotype 4, 5, or 6 infection. These results along with previously reported promising efficacy in GT1, 2, and 3 infection establish potent clinical pangenotypic activity of this RBV-free once daily Compound 1 + Compound 2 regimen.

### Example 16. High Efficacy and Favorable Safety of Compound 1 and Compound 2 Co-Administration for 12 Weeks in HCV Genotype 1-Infected Patients With Cirrhosis

This study evaluated the safety and efficacy of Compound 1 and Compound 2 administered for 12 weeks in HCV GT1-infected patients with compensated cirrhosis. Treatment-naïve or pegylated interferon/ribavirin treatment-experienced patients received Compound 1 200 mg + Compound 2 120 mg once daily for 12 weeks. Cirrhosis was determined by either liver biopsy (Metavir F4), Fibroscan (liver stiffness > 14.6 KPa) or serum markers (Fibrotest score ≥ 0.75 and an APRI > 2). SVR at post-treatment week 12 (SVR12; HCV RNA levels determined using Roche COBAS TaqMan® RT-PCR assay [lower limit of detection of 15 IU/mL and lower limit of quantification of 25 IU/mL]) and safety were assessed.

A total of 27 patients were enrolled and the population was 74% male, 89% white, 74% GT1a, 85% non-CC IL28B, 26% HCV treatment-experienced, and all reported fibrosis scores of F4 at baseline (1 missing). The median (range) HCV RNA log₁₀ IU/mL was 6.7 (5.6-7.3), and 93% had HCV RNA ≥ 6,000,000 IU/mL at baseline. Efficacy data shows 26 out of 27 (96%) of patients achieved SVR12, with one patient experiencing relapse at post-treatment week 4. Most adverse events (AEs) were deemed Grade 1 (mild) or Grade 2 (moderate) in severity, with no patients reporting severe or serious AEs considered related to study drugs. No patients discontinued treatment prematurely due to AEs and the most frequent AEs reported in >10% of patients were fatigue (11%) and headache (11%). While on-treatment, no clinically meaningful abnormal liver function or other laboratory results were observed.

The study showed that treatment with the IFN- and ribavirin-free combination of Compound 1 and Compound 2 was well-tolerated and achieved high SVR12 rates of 96% following a 12-week treatment regimen in GT1-infected patients with compensated cirrhosis regardless of baseline viral load or prior treatment history.

The foregoing description of the present invention provides illustration and description, but is not intended to be exhaustive or to limit the invention to the precise one disclosed. Modifications and variations are possible in light of the above teachings or may be acquired from practice of the invention. Thus, it is noted that the scope of the invention is defined by the claims and their equivalents.

## Claims

1. Compound 1 and Compound 2 for use in a method of treatment for hepatitis C virus (HCV) infection, wherein 200-600 mg Compound 1 or a pharmaceutically acceptable salt thereof is administered once daily, and 100-500 mg Compound 2 or a pharmaceutically acceptable salt thereof is administered once daily, to a patient infected with HCV, wherein said treatment does not include administration of either interferon or ribavirin to said patient, and said treatment lasts for 8, 12 or 16 weeks; and wherein Compound 1 is 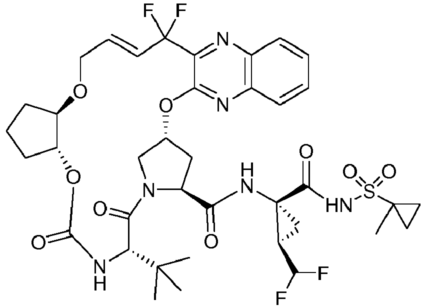 and Compound 2 is

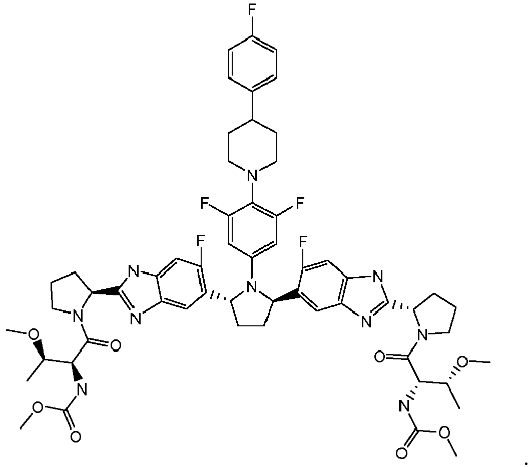

2. Compound 1 and Compound 2 for use according to claim 1, wherein said treatment lasts for 12 weeks.

3. Compound 1 and Compound 2 for use according to claim 1, wherein said treatment lasts for 8 weeks.

4. Compound 1 and Compound 2 for use according to claim 1, wherein said treatment lasts for 16 weeks.

5. Compound 1 and Compound 2 for use according to any of claims 1 to 4, wherein said patient is administered with 300 mg Compound 1 once daily and 120 mg Compound 2 once daily.

6. Compound 1 and Compound 2 for use according to any of claims 1 to 5, wherein said patient is without cirrhosis.

7. Compound 1 and Compound 2 for use according to any of claims 1 to 5, wherein said patient is with compensated cirrhosis.

8. Compound 1 and Compound 2 for use according to any of claims 1 to 5, wherein said patient is infected with HCV genotype 1.

9. Compound 1 and Compound 2 for use according to any of claims 1 to 5, wherein said patient is infected with HCV genotype 2.

10. Compound 1 and Compound 2 for use according to any of claims 1 to 5, wherein said patient is infected with HCV genotype 3.

11. Compound 1 and Compound 2 for use according to any of claims 1 to 5, wherein said patient is infected with HCV genotype 4.

12. Compound 1 and Compound 2 for use according to any of claims 1 to 5, wherein said patient is infected with HCV genotype 5.

13. Compound 1 and Compound 2 for use according to any of claims 1 to 5, wherein said patient is infected with HCV genotype 6.

14. Compound 1 and Compound 2 for use in a method of treatment for HCV infection, wherein 200-600 mg Compound 1 or a pharmaceutically acceptable salt thereof is administered once daily, and 100-500 mg Compound 2 or a pharmaceutically acceptable salt thereof is administered once daily, to a patient infected with HCV, wherein said treatment does not include administration of either interferon or ribavirin to said patient, and said treatment lasts for 16 weeks; and wherein Compound 1 is 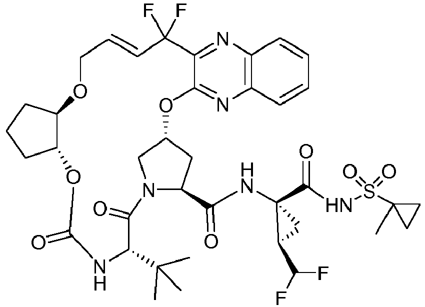 and Compound 2 is

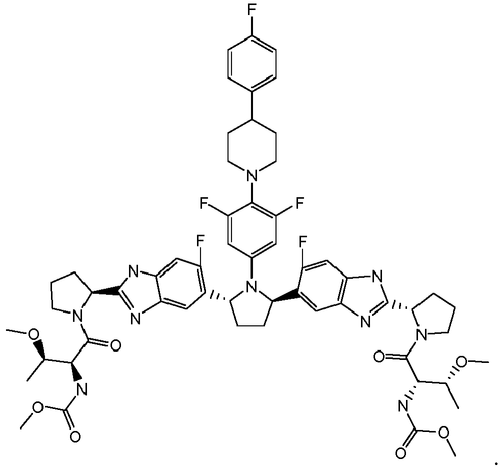

15. Compound 1 and Compound 2 for use according to claim 14, wherein said patient is administered with 300 mg Compound 1 once daily and 120 mg Compound 2 once daily.

16. Compound 1 and Compound 2 for use according to claim 14 or claim 15, wherein said patient is infected with HCV genotype 1.

17. Compound 1 and Compound 2 for use according to claim 14 or claim 15, wherein said patient is infected with HCV genotype 2.

18. Compound 1 and Compound 2 for use according to claim 14 or claim 15, wherein said patient is infected with HCV genotype 3.

19. Compound 1 and Compound 2 for use according to claim 14 or claim 15, wherein said patient is infected with HCV genotype 4.

20. Compound 1 and Compound 2 for use according to claim 14 or claim 15, wherein said patient is infected with HCV genotype 5.

21. Compound 1 and Compound 2 for use according to claim 14 or claim 15, wherein said patient is infected with HCV genotype 6.
